# EUROPEAN PATENT APPLICATION

(11) **EP 4 667 457 A1**
(43) Date of publication of application: **24.12.2025**
(21) Application number: 23922473.6
(22) Date of filing: 08.12.2023
(51) Int. Cl.: C07D 215/233, C07D 221/04, A61K 31/47, A61K 31/4709, A61P 5/14, A61P 9/10, A61P 3/06, A61P 1/16

(54) **POLYCYCLIC THYROID HORMONE BETA RECEPTOR AGONIST AND USE THEREOF**

(30) Priority: 17.02.2023 CN 202310134989; 16.11.2023 CN 202311537585
(71) Applicant: Cascade Pharmaceuticals, Inc., Shanghai 201422 (CN)
(72) Inventor: SHI, Jingjing, Shanghai 201422 (CN); YANG, Shengsheng, Shanghai 201422 (CN); GONG, Linpei, Shanghai 201422 (CN); WANG, Peng, Shanghai 201422 (CN); ZHU, Xin, Shanghai 201422 (CN)
(74) Representative: Hoefer & Partner Patentanwälte mbB
(86) International application number: PCT/CN2023/137413
(87) International publication number: WO 2024/169357

(57) **Abstract**

The present invention relates to a polycyclic thyroid hormone β receptor agonist and use thereof, in particular, to a compound represented by formula (1) or a pharmaceutically acceptable salt thereof, a pharmaceutical composition comprising same, a preparation method therefor, and use thereof. The compound or the pharmaceutical composition of the present invention can be used for preparing a drug for preventing, treating, or relieving diseases regulated by the thyroid hormone β receptor.

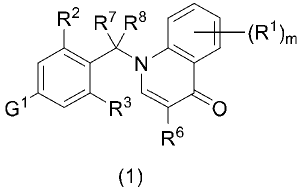

## Description

### CROSS-REFERENCE TO RELATED APPLICATION

The present application claims the priority to and the benefits of Chinese Patent Application No. 202310134989.4 filed with the National Intellectual Property Administration, PRC on February 17, 2023, and the priority to and the benefits of Chinese Patent Application No. 202311537585.6 filed with the National Intellectual Property Administration, PRC on November 16, 2023, the disclosures of which are incorporated herein by reference in their entirety.

### TECHNICAL FIELD

The present disclosure belongs to the field of pharmaceutical chemistry, and relates to a polycyclic compound as thyroid hormone β receptor agonist, a pharmaceutical composition comprising the same, a preparation method therefor, and use thereof in the preparation of a medicament for preventing, treating or alleviating a disease regulated by thyroid hormone β receptor.

### BACKGROUND

Thyroid hormone (TH) is synthesized in thyroid in response to the thyroid stimulating hormone (TSH) secreted by pituitary. Thyroid hormone plays a very important role in regulating body growth, development, metabolism, and homeostasis. Thyroid hormone exerts its functions by binding to thyroid hormone receptors (THRs). THRs belong to the nuclear receptor superfamily. Nuclear receptor, together with its common ligand - retinoid X receptor, forms a heterodimer that functions as a ligand-induced transcription factor. Like the other nuclear receptors, THRs have a ligand-binding domain and a DNA-binding domain, and regulate gene expression through their ligand-dependent interactions with the DNA response elements (thyroid hormone response elements, THREs).

There are currently two subtypes of THR: THRα and THRβ. THRα is mainly distributed in the heart tissue and plays an important regulatory role in the functions of the heart. THRβ is expressed primarily in the liver and hypophysis cerebri, regulates the metabolism of fatty acids and cholesterol, and regulates secretion of the thyroid stimulating hormone. Both THRα and THRβ are expressed in the brown adipose tissue (BAT), and play crucial roles in regulating basal oxygen consumption, fat storage, adipogenesis, and lipolysis (Oppenheimer et al., J. Clin. Invest. 87(1): 125-32 (1991)).

THR agonist may increase the metabolic rate, oxygen consumption, and thermogenesis, promote the metabolism of cholesterol into bile acids, and may additionally reduce the lipoprotein level associated with atherosclerosis. The liver and heart are the main target organs of the THR agonist. In the liver, the THR agonist primarily regulates the genes related to the synthesis and metabolism of fatty acids and cholesterol, and exerts impacts on carbohydrates by increasing glycogenolysis and glyconeogenesis and lowering the effect of insulin. In the heart, it may reduce systemic vascular resistance, increase blood volume, and generate inotropic and chronotropic effects.

THRβ agonist can also improve cellular lipid metabolism and exert the effects of lowering cholesterol and blood lipids. Therefore, it is of great significance to research and develop a THRβ agonist for treating and/or preventing diseases regulated by thyroid hormone receptors.

### SUMMARY

After extensive studies, the present disclosure has discovered a series of polycyclic compounds as thyroid hormone β receptor agonists having potential value in preventing and/or treating diseases regulated by thyroid hormone β receptors.

In the first aspect, the present disclosure provides a compound having a structure of formula (1) or a pharmaceutically acceptable salt thereof: 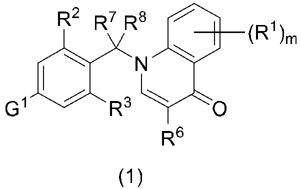 wherein
G¹ is 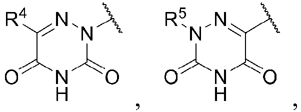 -O-(C₁₋₆ alkylene)-G², -NH-(C₁₋₆ alkylene)-G², -NHCO-(C₁₋₆ alkylene)-G² or -NHCO-G²;
G² is -COOH, -P(=O)(OH)₂, -P(-O)(OC₁₋₆ alkyl)₂, 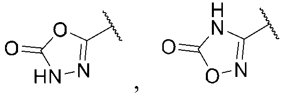 or 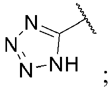
R⁴ and R⁵ are independently H, halogen, -CN, -NH₂, -NO₂, -OH, C₁₋₆ alkyl, -O(C₁₋₆ alkyl), -COO(C₁₋₆ alkyl), C₂₋₆ alkenyl, C₂₋₆ alkynyl or C₃₋₈ cycloalkyl; said C₁₋₆ alkyl, C₂₋₆ alkenyl, C₂₋₆ alkynyl or C₃₋₈ cycloalkyl is optionally substituted with one or more substituents that are independently deuterium, halogen, -CN, -NH₂, -NO₂ or -OH;
R² and R³ are independently H, halogen, -CN, -NH₂, -NO₂, -OH or C₁₋₆ alkyl; said C₁₋₆ alkyl is optionally substituted with one or more substituents that are independently halogen, -CN, -NH₂, -NO₂ or -OH;
R⁶ is H, -COOH, -COO(C₁₋₆ alkyl), -CONH(C₁₋₆ alkyl) or -CONH(C₃₋₈ cycloalkyl); said C₁₋₆ alkyl or C₃₋₈ cycloalkyl is optionally substituted with one or more substituents that are independently halogen, -CN, -NH₂, -NO₂ or -OH;
R⁷ and R⁸ are independently H or deuterium;
each R¹ is independently H, halogen, -CN, -NH₂, -NO₂, -OH, C₁₋₆ alkyl, -O(C₁₋₆ alkyl) or -COO(C₁₋₆ alkyl); said C₁₋₆ alkyl is optionally substituted with one or more substituents that are independently halogen, -CN, -NH₂, -NO₂ or -OH; and
m is 0, 1, 2, 3 or 4.

In some embodiments, G¹ is 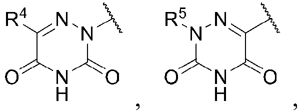 -O-(C₁₋₃ alkylene)-G², -NH-(C₁₋₃ alkylene)-G², -NHCO-(C₁₋₃ alkylene)-G² or -NHCO-G²; and
G² is -COOH, -P(=O)(OH)₂, -P(=O)(OC₁₋₃ alkyl)₂, 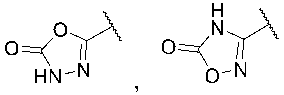 or

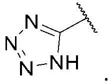

In some preferred embodiments, G¹ is

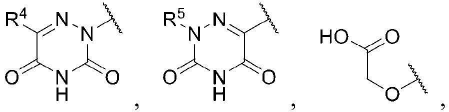

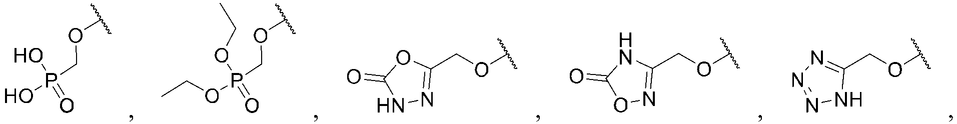

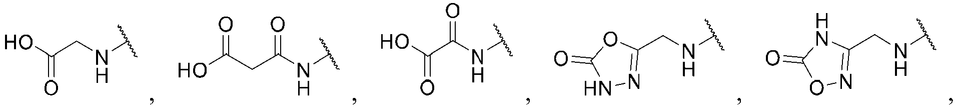

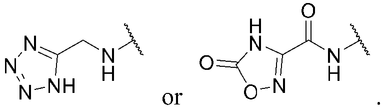

In some embodiments, R⁴ and R⁵ are independently H, halogen, -CN, -NH₂, -NO₂, -OH, C₁₋₃ alkyl, -O(C₁₋₃ alkyl), -COO(C₁₋₃ alkyl), C₂₋₄ alkenyl, C₂₋₄ alkynyl or C₃₋₆ cycloalkyl; said C₁₋₃ alkyl, C₂₋₄ alkenyl, C₂₋₄ alkynyl or C₃₋₆ cycloalkyl is optionally substituted with one or more substituents that are independently deuterium, halogen, -CN, -NH₂, -NO₂ or -OH.

In some preferred embodiments, R⁴ and R⁵ are independently H, -CN, -NH₂, -CH₃, -CH₂CH₃, -CH₂F, -CDF₂, -CHF₂, -CF₃, -COOCH₃, -CH₂OH, -CH=CH₂, -OCH₃, -C=CH or

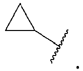

In some embodiments, R² and R³ are independently H, F, Cl, Br, -CN, -NH₂ or C₁₋₃ alkyl; said C₁₋₃ alkyl is optionally substituted with one or more substituents that are independently F, Cl, Br, -CN, -NH₂, -NO₂ or -OH.

In some preferred embodiments, R² and R³ are independently H, F, Cl, Br or -CH₃.

In some embodiments, R⁶ is H, -COOH, -COO(C₁₋₃ alkyl), -CONH(C₁₋₃ alkyl) or -CONH(C₃₋₆ cycloalkyl); said C₁₋₃ alkyl or C₃₋₆ cycloalkyl is optionally substituted with one or more substituents that are independently halogen, -CN, -NH₂, -NO₂ or -OH.

In some preferred embodiments, R⁶ is H, -COOH, -COOCH₃, -COOCH₂CH₃, -CONHCH₃,

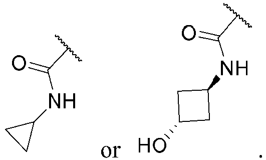

In some embodiments, each R¹ is independently H, halogen, -CN, -NH₂, -NO₂, -OH, C₁₋₃ alkyl, -O(C₁₋₃ alkyl) or -COO(C₁₋₃ alkyl); said C₁₋₃ alkyl is optionally substituted with one or more substituents that are independently halogen, -CN, -NH₂, -NO₂ or -OH.

In some preferred embodiments, each R¹ is independently H, F, Cl, Br, -CN, -CH₃, -CF₃, -OCH₃, -OCF₃ or -COOCH₃.

In some embodiments, both R⁷ and R⁸ are deuterium.

In some embodiments, either of R⁷ and R⁸ is H, and the other is deuterium.

In some embodiments, m is 0, 1 or 2.

In some embodiments, the present disclosure provides a compound having a structure of any one of formula (2) to formula (7) or a pharmaceutically acceptable salt thereof: 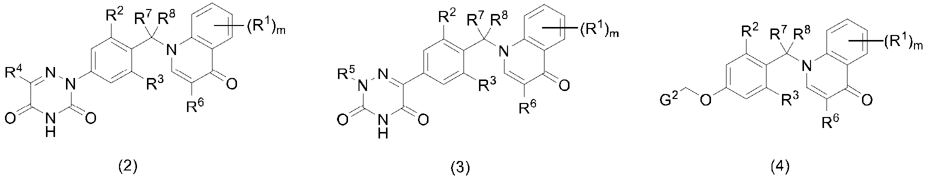 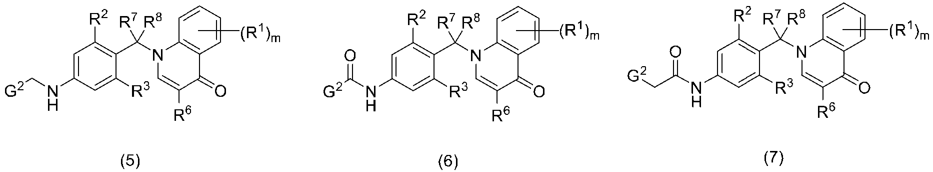 wherein G², R¹, R², R³, R⁴, R⁵, R⁶, R⁷, R⁸, and m are as defined in formula (1).

A person skilled in the art shall appreciate that the present disclosure encompasses the compounds obtained by arbitrarily combining various embodiments. The embodiments obtained by combining the technical features or preferred technical features of one embodiment with the technical features or preferred technical features of another embodiment are also included within the scope of the present disclosure.

In the second aspect, the present disclosure further provides the following compounds, or pharmaceutically acceptable salts thereof:

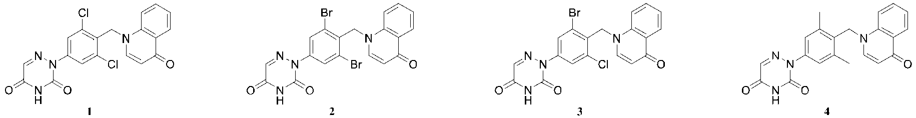

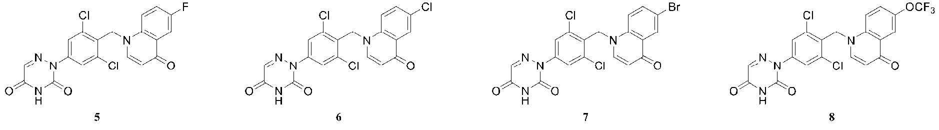

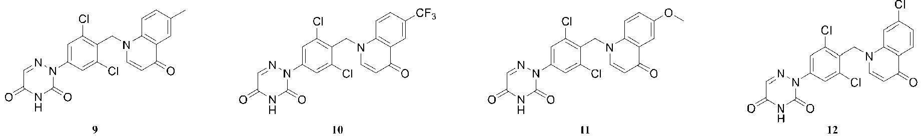

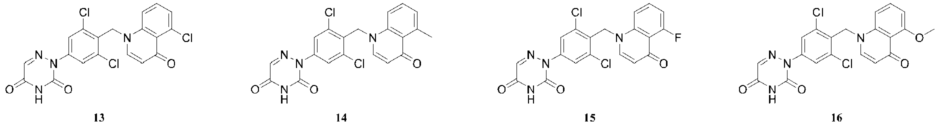

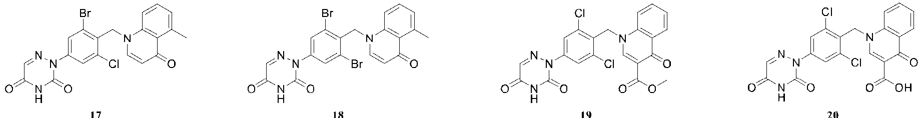

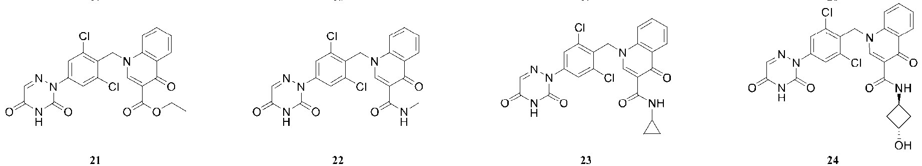

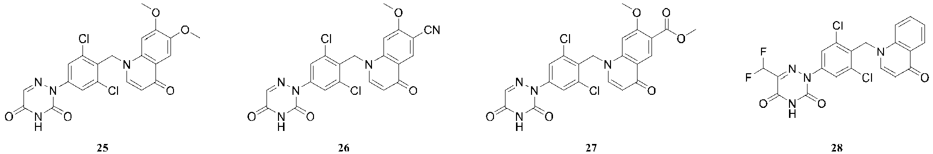

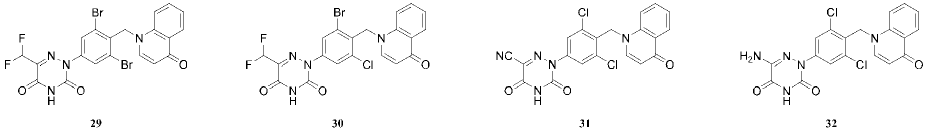

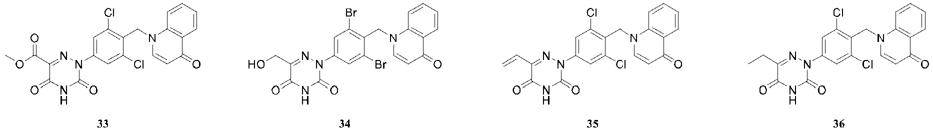

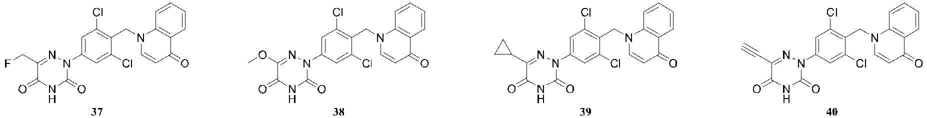

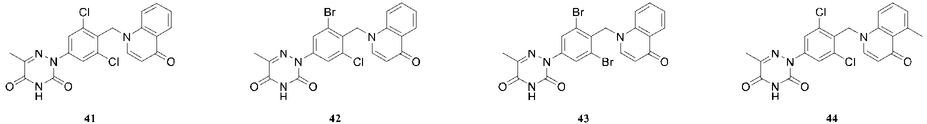

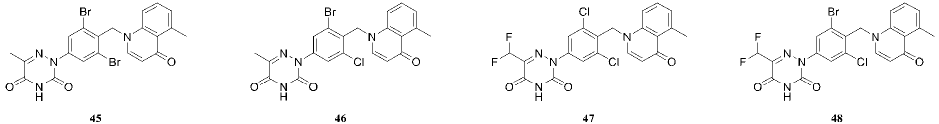

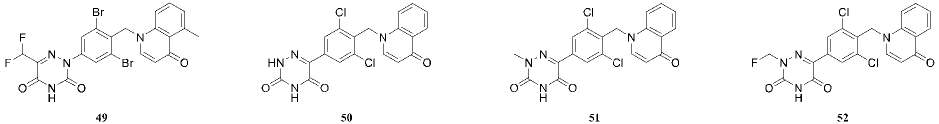

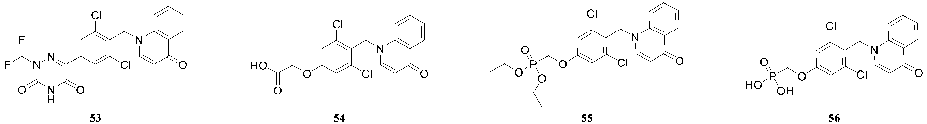

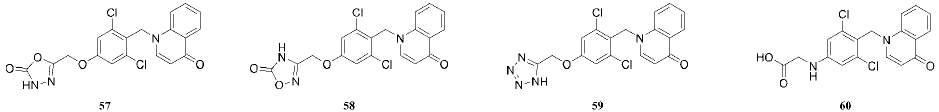

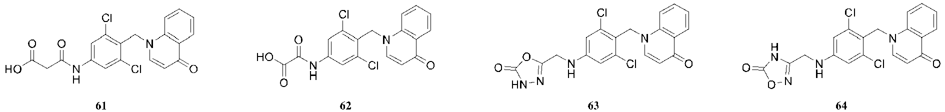

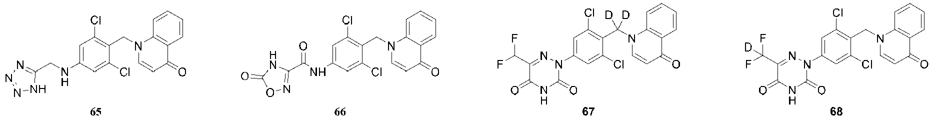

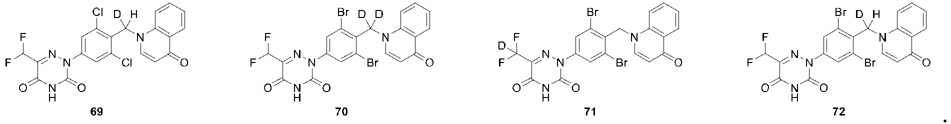

In the third aspect, the present disclosure provides a method for preparing a compound of formula (2), comprising the following steps:

### Step 1: Synthesis of Intermediate M

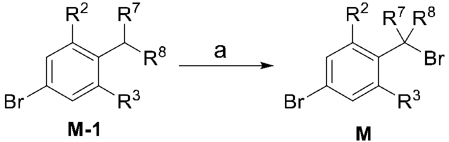

(a) A compound represented by general formula **M-1** is used as a starting material and reacts under the action of N-bromosuccinimide and a radical initiator to afford a compound represented by general formula **M.**

In some embodiments, (a) in the above step 1 is conducted in the presence of a radical initiator selected from the group consisting of azobisisobutyronitrile, azobisisoheptonitrile, cumene hydroperoxide, tert-butyl hydroperoxide, p-menthane hydroperoxide, dibenzoyl peroxide, dodecanoyl peroxide, di-tert-butyl peroxide, and dicumyl peroxide, preferably azobisisobutyronitrile.

### Step 2: Synthesis of Compound Represented by Formula (2)

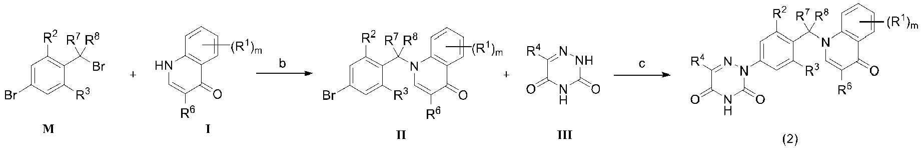

(b) the compound represented by general formula **M** is used as a starting material and reacts with a compound represented by general formula **I** under the action of a base to afford a compound represented by general formula **II;**
(c) the compound represented by general formula **II** and the compound represented by general formula **III** react with a copper catalyst under the action of a base to afford a compound represented by formula (2).

In some embodiments, (b) in the above step 2 is conducted in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium hydroxide, sodium methoxide, sodium ethoxide, potassium ethoxide, potassium acetate, and sodium acetate, preferably potassium carbonate.

In some embodiments, (c) in the above step 2 is conducted in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium phosphate, potassium hydroxide, sodium methoxide, sodium ethoxide, and potassium ethoxide, preferably potassium phosphate and potassium carbonate.

In some embodiments, (c) in the above step 2 is conducted in the presence of a copper catalyst selected from the group consisting of cuprous oxide, cuprous chloride, cuprous iodide, cuprous thiocyanate, copper acetate, cuprous bromide, copper, copper oxide, copper chloride, copper bromide, and copper iodide, preferably cuprous iodide.

In the fourth aspect, the present disclosure provides a pharmaceutical composition, comprising at least one of the compounds or pharmaceutically acceptable salts thereof as described above, and one or more pharmaceutically acceptable carriers.

In the fifth aspect, the present disclosure provides the compounds or pharmaceutically acceptable salts thereof as described above, or the pharmaceutical composition as described above, as thyroid hormone β receptor agonist, for use in prevention and/or treatment of a disease or condition mediated by thyroid hormone β receptor.

In the sixth aspect, the present disclosure provides use of the compounds or pharmaceutically acceptable salts thereof as described above, or the pharmaceutical composition as described above in the preparation of a medicament for preventing and/or treating a disease or condition mediated by thyroid hormone β receptor (for example, a metabolic disease such as nonalcoholic fatty liver disease, dyslipidemia, atherosclerosis or hypothyroidism).

In the seventh aspect, the present disclosure provides a method for preventing and/or treating a disease or condition mediated by thyroid hormone β receptor, comprising a step of administering, to a subject in need thereof, a prophylactically and/or therapeutically effective amount of the compounds or pharmaceutically acceptable salts thereof as described above, or the pharmaceutical composition as described above.

The present disclosure is not limited to the particular embodiments described herein. It should also be understood that the terms used herein are intended only to describe rather than limit the particular embodiments.

### Definitions of Terms

Unless otherwise specified, the meanings of the following terms in the present disclosure are as follows:
The term "including", "comprising", "having" or "containing" or any other variant thereof is intended to encompass non-exclusive or open-ended inclusions. For example, a composition, method or apparatus including a series of elements is not necessarily limited only to the elements that have been explicitly recited, and may further include other elements that are not explicitly recited or the elements innate in the above composition, method or apparatus.

When the lower limit and the upper limit of a numerical range are disclosed, it means that any value or sub-range within this range is specifically disclosed. In particular, each of the numerical ranges (*e.g.,* in the form of "from about a to b", or equivalently "from approximately a to b", or equivalently "about a-b") of the parameters disclosed herein is to be understood to encompass each of the values and sub-ranges therein. For example, "C₁₋₄" should be understood to encompass any sub-range and each point value therein, *e.g.,* C₂₋₄, C₃₋₄, C₁₋₂, C₁₋₃, or C₁₋₄, etc., and C₁, C₂, C₃, C₄, etc. For another example, "5- to 10-membered" should be understood to encompass any sub-range and each point value therein, *e.g.,* 5- to 6-membered, 5- to 7-membered, 5- to 8-membered, 5- to 9-membered, 6- to 7-membered, 6- to 8-membered, etc., and 5-, 6-, 7-, 8-, 9-, 10-membered, etc.

The term "substitute" and other variations thereof used herein mean that one or more (such as 1, 2, 3, or 4) atoms or atomic groups (such as a hydrogen atom) linked to the designated atom are replaced with additional equivalent(s), provided that the valence of the designated atom or atomic group in the current situation can be kept normal and that a stable compound can be formed. If an atom or atomic group is described as being "optionally substituted with ...", it may be either substituted or unsubstituted. Unless otherwise specified, a linking position of a substituent described herein may be at any appropriate position of the substituent. When a linking bond in a substituent is shown as a chemical bond across two interconnected atoms in a ring system, it means that the substituent may be linked to any of the ring atoms in the ring system.

The term "pharmaceutical composition" refers to a composition that can be used as a medicament, comprising an active pharmaceutical ingredient (or a therapeutic agent) and one or more optional pharmaceutically acceptable carriers. The term "pharmaceutically acceptable carrier" refers to an excipient administered together with a therapeutic agent, which is suitable, within the scope of reasonable medical judgment, for contact with the tissue of human beings and/or other animals without undue toxicity, irritation, allergic response or other problems or complications commensurate with a reasonable benefit/risk ratio. Pharmaceutically acceptable carriers usable in the present disclosure include, but are not limited to, a) diluents; b) lubricants; c) binders; d) disintegrants; e) absorbents, colorants, flavoring agents, and/or sweetening agents; f) emulsifiers or dispersants; and/or g) substances that enhance absorption of the compounds.

The above-mentioned pharmaceutical compositions may act systemically and/or topically. To this end, they may be administered via suitable routes, *e*.*g*., parenteral, topical, intravenous, oral, subcutaneous, intra-arterial, intradermal, transdermal, rectal, intracranial, intraperitoneal, intranasal, or intramuscular route, or administered as inhalants.

The routes of administration as described above can be achieved by suitable dosage forms. The dosage form usable in the present disclosure includes, but is not limited to, tablets, capsules, troches, dragees, powders, sprays, emulsions, ointments, suppositories, gels, pastes, lotions, ointments, aqueous suspensions, injectable solutions, elixirs, syrups, etc.

When administered orally, the above-mentioned pharmaceutical composition may be formulated into any orally acceptable dosage form, including but not limited to, tablets, capsules, aqueous solutions, aqueous suspensions, etc.

The above-mentioned pharmaceutical composition may also be administered in the form of a sterile injectable preparation including sterile injectable aqueous or oily suspensions or sterile injectable aqueous or oily solutions. The carriers that can be used include, but are not limited to, water, Ringer's solution, and isotonic sodium chloride solution. In addition, sterile non-volatile oils may also be used as solvents or suspension media, such as monoglycerides or diglycerides.

The above-mentioned pharmaceutical composition may comprise 0.01 mg to 1000 mg of at least one of the above compounds of formula (1) to formula (7) or pharmaceutically acceptable salts thereof.

The term "disease or condition mediated by thyroid hormone β receptor" refers to the disease whose pathogenesis involves at least a part of factors associated with the thyroid hormone β receptor, *e.g.,* a metabolic disease such as nonalcoholic fatty liver disease, dyslipidemia, atherosclerosis or hypothyroidism.

The term "effective amount" refers to a dose capable of inducing a biological or medical response in a cell, tissue, organ, or organism (*e*.*g*., a subject) and is sufficient to achieve the desired prophylactic and/or therapeutic effect.

The dosage regimen may be adjusted to provide an optimal response. For example, a single dose may be administered, divided doses may be administered over time, or the dose may be administered after it is proportionally reduced or increased as appropriate. It should be appreciated that for any particular subject, the specific dosage regimen should be adjusted according to the needs and the professional judgment of the practitioner who administers the composition or supervising administration of the composition.

The term "in need thereof" refers to a judgment made by a doctor or other paramedics on a subject who needs to or will benefit from the course of prevention and/or treatment, and the judgment is made based on various factors in the doctor or other paramedics' areas of expertise.

The term "subject" (or referred to as testee) refers to human beings or non-human animals. The subject of the present disclosure includes a subject (patient) with a disease and/or a condition and a normal subject. The non-human animals of the present disclosure include all vertebrates, *e.g.,* non-mammals such as birds, amphibians, and reptiles, and mammals, *e.g.,* non-human primates, livestock, and/or domesticated animals (such as sheep, dogs, cats, cows, and pigs).

The term "treatment" refers to alleviation or elimination of a target disease or condition. If a testee receives a therapeutic dose of the compound of the present disclosure or a pharmaceutically acceptable salt thereof or the pharmaceutical composition of the present disclosure and at least one index or symptom of the testee shows observable and/or detectable alleviation and/or amelioration, this indicates that the testee has been successfully "treated". It is to be appreciated that the treatment includes not only complete treatment but also achievement of some biologically or medically relevant outcomes while not yet completing treatment. Specifically, "treatment" means that the compound of the present disclosure or a pharmaceutically acceptable salt thereof or the pharmaceutical composition of the present disclosure can achieve at least one of the following effects: for example, (1) prevention of diseases occurring in animals that may be predisposed to diseases but have not yet experienced or showed pathological or symptomatological features of the diseases; (2) suppression of diseases (*i.e*., prevention of further development in pathology and/or symptomatology) in animals that are experiencing or showing pathological or symptomatological features of the diseases; and (3) amelioration of diseases (*i.e*., disease reversion in pathology and/or symptomatology) in animals that are experiencing or showing pathological or symptomatological features of the diseases.

The term "pharmaceutically acceptable salt" refers to salts of the compounds of the present disclosure that are substantially non-toxic to an organism. Pharmaceutically acceptable salts generally include, but are not limited to, salts formed by reacting the compounds of the present disclosure with a pharmaceutically acceptable inorganic/organic acid or inorganic/organic base, and such salts are also referred to as acid addition salts or base addition salts. For a review of suitable salts, please see, for example, Jusiak, Soczewinski, et al., Remington's Pharmaceutical Sciences [M], Mack Publishing Company, 2005 and Stahl, Wermuth, Handbook of Pharmaceutical Salts: Properties, Selection, and Use [M], Wiley-VCH, 2002. Methods for preparing pharmaceutically acceptable salts of the compounds of the present disclosure are known to a person skilled in the art.

The term "pharmaceutically acceptable ester" refers to esters substantially non-toxic to an organism and hydrolyzed in the organism into the compounds of the present disclosure or salts thereof. Pharmaceutically acceptable esters generally include, but are not limited to, esters formed by reacting the compounds of the present disclosure with a pharmaceutically acceptable carboxylic acid or sulfonic acid, and such esters are also known as carboxylic acid esters or sulfonic acid esters.

The term "isomer" refers to the compounds that have the same molecular weight because of the same number and type of atoms, but differ in the spatial arrangement or configuration of the atoms.

The term "stereoisomer" (or "optical isomer") refers to a stable isomer having a perpendicular and asymmetric plane due to the presence of at least one chiral element (including a chiral center, a chiral axis, and a chiral plane), thereby enabling rotation of plane-polarized light. Since the compounds of the present disclosure have asymmetric centers and other chemical structures that may lead to stereoisomerism, the present disclosure also includes these stereoisomers and mixtures thereof. Unless otherwise indicated, all stereoisomeric forms of the compounds of the present disclosure are within the scope of the present disclosure.

The term "tautomer" (or "tautomeric form") refers to structural isomers with different energies that may be interconvertible through a low energy barrier. If tautomerism is possible (*e.g*., in a solution), a chemical equilibrium of tautomers can be achieved. For example, the causes of proton tautomers (or prototropic tautomers) include, but are not limited to, interconversion via proton migration, such as keto-enol tautomerism, imine-enamine tautomerism, and amide-iminol tautomerism. Unless otherwise indicated, all tautomeric forms of the compounds of the present disclosure are within the scope of the present disclosure.

The term "solvate" refers to a substance formed by the association of the compound of the present disclosure (or a pharmaceutically acceptable salt thereof) with at least one kind of solvent molecule through non-covalent intermolecular forces. For example, the solvate includes, but is not limited to, hydrates (including hemihydrates, monohydrates, dihydrates, trihydrates, etc.), ethanolates, and acetoneates.

The term "nitrogen oxide" refers to a compound formed by oxidation of a nitrogen atom in the structure of a tertiary amine or nitrogen-containing (aromatic) heterocyclic compound. For example, the nitrogen atom in the parent nucleus of the compound of formula I can form the corresponding nitrogen oxide.

The term "isotope-labelled compound" refers to a derivative compound formed by replacing a particular atom in the compound of the present disclosure with its isotopic atom. Unless otherwise indicated, the compounds of the present disclosure include various isotopes of H, C, N, O, F, P, S, and Cl, for example, but not limited to ²H(D), ³H(T), ¹³C, ¹⁴C, ¹⁵ N, ¹⁷O, ¹⁸O, ¹⁸F, ³¹P, ³²P, ³⁵S, ³⁶S, and ³⁷Cl.

The term "metabolite" refers to a derivative compound formed after the compound of the present disclosure is metabolized. For further information on metabolism, please see Goodman and Gilman's: The Pharmacological Basis of Therapeutics (9th ed.) [M], McGraw-Hill International Editions, 1996. The present disclosure encompasses all possible metabolite forms of the compounds of the present disclosure, *i.e.,* substances formed in the body of a subject administered with the compound of the present disclosure. Metabolites of the compounds can be identified by techniques well known in the art, and their activities can be experimentally characterized.

The term "prodrug" refers to a derivative compound that, after administered to a subject, is capable of offering, directly or indirectly, the compound of the present disclosure. Particularly preferred derivative compounds or prodrugs are the compounds that, when administered to a subject, can increase the bioavailability of the compounds of the present disclosure (*e*.*g*., more readily absorbed into the blood), or the compounds that facilitate delivery of the parent compound to the site of action (*e.g*., the lymphatic system). Unless otherwise indicated, all prodrug forms of the compounds of the present disclosure are within the scope of the present disclosure, and various prodrug forms are known in the art, see, *e.g.,* T. Higuchi, V. Stella, Pro-drugs as Novel Drug Delivery Systems [J], American Chemical Society, Vol. 14, 1975**.** In addition, the present disclosure further encompasses the compounds of the present disclosure containing a protective group. In any process of preparing the compounds of the present disclosure it may be necessary and/or desirable to protect sensitive or reactive groups on any of the molecules concerned, thereby forming a chemically protected form of the compounds of the present disclosure. This may be realized by conventional protective groups, such as those described in T. W. Greene, P. G. M. Wuts, Greene's Protective Groups in Organic Synthesis [M], John Wiley & Sons, 2006**.** These protective groups may be removed at an appropriate subsequent stage by the methods known in the art.

The term "independently" means that at least two groups (or ring systems) with the same or similar range in the structure may have the same or different meanings under particular circumstances. For example, if the substituent X and the substituent Y are independently hydrogen, halogen, hydroxyl, cyano, alkyl, or aryl, when the substituent X is hydrogen, the substituent Y may either be hydrogen or be halogen, hydroxyl, cyano, alkyl, or aryl; similarly, when the substituent Y is hydrogen, the substituent X may either be hydrogen or be halogen, hydroxyl, cyano, alkyl, or aryl.

When used herein alone or in combination with an additional group, the term "halogen" refers to fluorine (F), chlorine (Cl), bromine (Br), and iodine (I).

When used herein alone or in combination with an additional group, the term "alkyl" refers to linear or branched aliphatic hydrocarbyl. For example, the term "C₁₋₆ alkyl" used in the present disclosure refers to alkyl having 1 to 6 carbon atoms. For example, alkyl may be methyl, ethyl, n-propyl, isopropyl, n-butyl, sec-butyl, isobutyl, or tert-butyl.

When used herein alone or in combination with an additional group, the term "cycloalkyl" refers to saturated monocyclic or polycyclic (such as bicyclic, *e.g.,* fused rings, bridged rings, or spiro rings) nonaromatic hydrocarbonyl. For example, the term "C₃₋₈ cycloalkyl" used in the present disclosure refers to cycloalkyl having 3 to 8 carbon atoms. For example, cycloalkyl may be cyclopropyl, cyclobutyl, cyclopentyl, cyclohexyl, or bicyclo[2.2.1]heptyl. Cycloalkyl in the present disclosure is optionally substituted with one or more substituents described herein.

When used herein alone or in combination with an additional group, the term "alkylene" refers to divalent, linear or branched, saturated aliphatic hydrocarbyl, in which the two groups (or fragments) it links may be linked either to the same carbon atom or to different carbon atoms. For example, the term "C₁₋₄ alkylene" refers to alkylene having 1 to 4 carbon atoms (such as methylene, 1,1-ethylene, 1,2-ethylene, 1,2-propylene, or 1,3-butylene).

When used herein alone or in combination with an additional group, the term "alkenyl" refers to linear or branched aliphatic hydrocarbyl having at least one C=C double bond. For example, "C₂₋₆ alkenyl" refers to alkenyl having 2 to 6 carbon atoms. Common alkenyl includes, but is not limited to, ethenyl, propenyl, n-butenyl, 3-methylbut-2-enyl, n-pentenyl, n-octenyl, n-decenyl, etc.

When used herein alone or in combination with an additional group, the term "alkynyl" refers to linear or branched aliphatic hydrocarbyl having at least one C≡C triple bond. For example, "C₂₋₆ alkynyl" refers to alkynyl having 2 to 6 carbon atoms. Common alkynyl includes, but is not limited to, ethynyl, 2-propynyl, 2-butynyl, 1,3-butadiynyl, etc.

### DETAILED DESCRIPTION

In order to render the purposes and technical solutions of the present disclosure clearer, the embodiments of the present disclosure will be described in detail below with reference to the examples. A person skilled in the art will appreciate, however, that the following examples are intended only to illustrate the present disclosure, and should not be considered to limit the scope of the present disclosure.

The reagents or instruments used in the examples are all commercially-available conventional products. Where no specific conditions are specified, conventional conditions or those recommended by the manufacturers are followed. The term "room temperature" used herein refers to 20°C±5°C. When used to modify a value or a numerical range, the term "about" used herein is meant to include this value or numerical range and an error range of this value or numerical range acceptable to a person skilled in the art, for example, the error range is ±10%, ±5%, ±4%, ±3%, ±2%, ±1%, or ±0.5%.

The structures of the compounds described in the examples below are determined by nuclear magnetic resonance (NMR) and/or mass spectrometry (MS).

The measuring instrument for the nuclear magnetic resonance (NMR) is a Bruker 400 MHz nuclear magnetic resonance instrument. Solvents for assay are deuterated methanol (CD₃OD), deuterated chloroform (CDCl₃), and dimethyl sulfoxide-*d*₆ (DMSO-*d*₆). The internal standard is tetramethylsilane (TMS). In ¹H NMR, part of the hydrogen may not show peaks as a result of the interference from salts or solvents.

The abbreviations used for the nuclear magnetic resonance (NMR) data in the following examples have the following meanings:
s: singlet, d: doublet, t: triplet, q: quartet, dd: double doublet, qd: quartet doublet, ddd: double double doublet, ddt: double double triplet, dddd: double double double doublet, m: multiplet, br: broad, *J*: coupling constant, Hz: Hertz, *δ*: chemical shift.

All chemical shift (δ) values are given in parts per million (ppm).

The measuring instrument for mass spectrometry is an Agilent 6120B mass spectrometer with an ion source being an electrospray ion (ESI) source.

HPLC assay uses Agilent 1200 DAD high pressure liquid chromatograph (Sunfire C18, 150 × 4.6 mm, 5 µm chromatographic column) and Waters 2695-2996 high pressure liquid chromatograph (Gimini C18, 150 × 4.6 mm, 5 µm chromatographic column).

Silica gel plates for thin layer chromatography are GF254 silica gel plates (Qingdao Haiyang Chemical Co., Ltd.). The silica gel plates used for the thin layer chromatography (TLC) have a thickness of 0.15 mm to 0.2 mm, and products are separated and purified by the thin layer chromatography using silica gel plates having a thickness of 0.4 mm to 0.5 mm.

Column chromatography generally uses 200 to 300-mesh silica gel (Qingdao Haiyang Chemical Co., Ltd.) as a carrier.

In the Examples, the reaction progress is monitored by thin layer chromatography (TLC). The system of the developing solvents used for the reactions includes A: dichloromethane - methanol system; and B: petroleum ether - ethyl acetate system. The volume ratio of solvents may be adjusted depending on the chemical polarity of the compounds.

The system of eluents for the normal phase column chromatography for purification of compounds and the system of the developing solvents for the thin layer chromatography include A: dichloromethane - methanol system; and B: petroleum ether - ethyl acetate system. The system of eluents for the reverse-phase column chromatography and the system of the developing solvents for the thin layer chromatography include A: water - methanol system; B: water - acetonitrile system. The volume ratio of solvents may be adjusted depending on the chemical polarity of the compounds, or adjusted by adding a small amount of triethylamine and acid or alkaline reagents, etc.

### Synthesis of Compounds

### Synthesis of Intermediate M1:

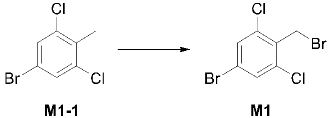

Compound **M1-1** (2.5 g, 10.4 mmol), N-bromosuccinimide (5.56 g, 32.1 mmol), and azobisisobutyronitrile (854 mg, 5.21 mmol) were added to carbon tetrachloride (40 mL), heated to 80°C, and stirred overnight. Upon completion of the reaction, the reaction solution was directly concentrated, and then purified by reverse-phase column chromatography [acetonitrile/water (containing 0.05% aqueous ammonia) = 0% to 100%] to afford compound **M1** (3.3 g, yield: 99.4%) as a white solid. MS (ESI, m/z): 318.8[M+H]⁺. ¹H NMR (400 MHz, DMSO-d6) δ 7.86 (s, 2H), 4.72 (s, 2H).

### Synthesis of Intermediate M3:

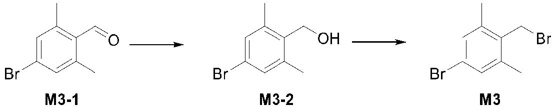

To a solution of compound **M3-1** (725 mg, 3.4 mmol) in methanol (7 mL), sodium borohydride (142 mg, 3.74 mmol) was added, and reacted at room temperature for 16 hours. The reaction solution was quenched with hydrochloric acid (1M, 50 mL), and extracted with ethyl acetate (50 mL×2). The organic phase was dried over anhydrous sodium sulfate, concentrated, and then purified by normal phase column chromatography (ethyl acetate: petroleum ether = 0% to 50%) to afford compound **M3-2** (730.2 mg, yield: 99.0%) as a yellow solid. MS (ESI, m/z): 214.8 [M+H]⁺.

To a solution of compound **M3-2** (730 mg, 3.4 mmol) in dichloromethane (7 mL), phosphorus tribromide (1.0 g, 3.74 mmol) was added dropwise. The reaction solution was stirred at room temperature for 2 hours. The reaction solution was directly concentrated, and then purified by reverse-phase column chromatography [acetonitrile/water (containing 0.05% aqueous ammonia) = 0% to 100%] to afford compound **M3** (935.9 mg, yield: 99.1%) as a white solid. MS (ESI, m/z): 278.8 [M+H]⁺.

¹H NMR (400 MHz, DMSO-d6) δ 7.36 (s, 2H), 4.86 (s, 2H), 2.23 (s, 6H).

### Example 1: Synthesis of Compound 1

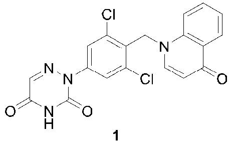

### Synthetic route:

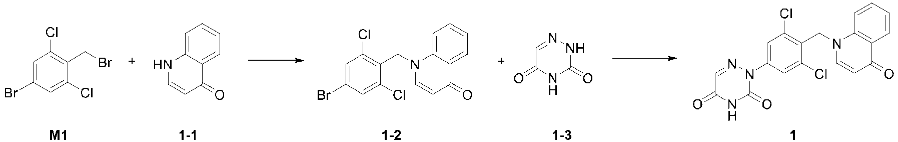

Compound **1-1** (500 mg, 3.45 mmol) was dissolved into N,N-dimethylformamide DMF (5 mL). Thereafter, sodium hydride (152 mg, 3.79 mmol) was added to the reaction solution at 0°C and stirred at 0°C for half an hour, and then compound **M1** (1.09 g, 3.45 mmol) was added thereto and stirred for two hours. After quenched with diluted hydrochloric acid (1 mol/L, 100 mL), the reaction system was extracted with ethyl acetate. The organic layer was concentrated and then purified by normal phase column chromatography (ethyl acetate: petroleum ether = 0% to 50%) to afford compound **1-2** (800.2 mg, yield: 61.5%). MS (ESI, m/z): 381.8 [M+H]⁺.

Compound **1-2** (100 mg, 0.28 mmol) and compound **1-3** (31 mg, 0.28 mmol) were dissolved into N,N-dimethylformamide (3 mL) solution. Thereafter, cuprous iodide (52 mg, 0.28 mmol), potassium phosphate (117 mg, 0.55 mmol), and N,N'-dimethylethylenediamine (24 mg, 0.28 mmol) were added, and heated to 120°C and reacted for two hours under nitrogen protection. Upon completion of the reaction, the reaction solution was directly concentrated, and then purified by reverse-phase column chromatography [acetonitrile/water (containing 0.05% aqueous ammonia) = 0% to 100%] to afford compound **1** (14.9 mg, yield: 11.3%) as a white solid. MS (ESI, m/z): 414.8 [M+H]⁺.

¹H NMR (400 MHz, DMSO-d6) δ 12.52 (s, 1H), 8.21 (d, *J* = 8.0 Hz, 1H), 7.84 - 7.80 (m, 3H), 7.77 (d, *J* = 7.2 Hz, 1H), 7.73 (s, 1H), 7.56 (d, *J =* 8.0 Hz, 1H), 7.42 (t, *J* = 7.2 Hz, 1H), 6.07 (d, *J* = 8.0 Hz, 1H), 5.65 (s, 2H).

### Example 2: Synthesis of Compound 2

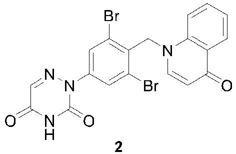

### Synthetic route:

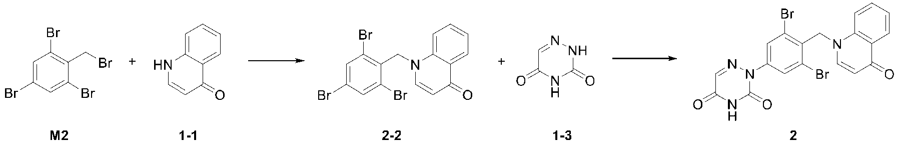

The synthetic method for intermediate **M1** was followed to afford intermediate **M2,** and subsequently the synthetic route of compound **1** was followed to synthesize compound **2,** which was then purified by reverse-phase column chromatography [acetonitrile/water (containing 0.05% aqueous ammonia) = 0% to 100%] to afford a yellow solid (15.2 mg, yield: 11.2%). MS (ESI, m/z): 504.8 [M+H]⁺.

¹H NMR (400 MHz, DMSO-*d*6) δ 8.22 (d, *J* = 6.8 Hz, 1H), 8.03 (s, 2H), 7.95 (d, *J* = 7.6 Hz, 1H), 7.85 - 7.66 (m, 2H), 7.53 - 7.41 (m, 1H), 7.32 (d, *J* = 7.2 Hz, 1H), 6.05 (d, *J* = 7.2 Hz, 1H), 5.60 (s, 2H).

### Example 3: Synthesis of Compound 3

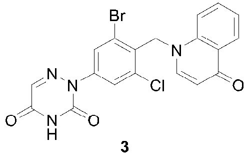

### Synthetic route:

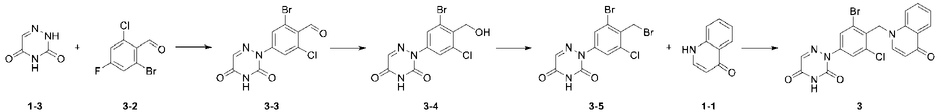

A mixture of compound **3-2** (200 mg, 0.844 mmol), compound **1-3** (114 mg, 1.013 mmol), potassium carbonate (174 mg, 1.27 mmol), and DMF (4 mL) was stirred at 80°C for 16 hours. The reaction solution was adjusted with hydrochloric acid to pH=2-3, diluted with water (30 mL), and extracted with ethyl acetate (30 mL×2). The organic phase was dried over anhydrous sodium sulfate, concentrated, and then purified by normal phase column chromatography (ethyl acetate: petroleum ether = 0% to 50%) to afford compound **3-3** (199.7 mg, yield: 70.1%) as a yellow solid. MS (ESI, m/z): 329.8 [M+H]⁺.

To a solution of compound **3-3** (200 mg, 0.604 mmol) in methanol (1 mL) and tetrahydrofuran (3 mL), sodium borohydride (25 mg, 0.664 mmol) was added, and reacted at room temperature for 1 hour. The reaction solution was quenched with hydrochloric acid (1M, 3 mL), diluted with water (30 mL), and extracted with ethyl acetate (30 mL×2). The organic phase was dried over anhydrous sodium sulfate, concentrated, and then purified by normal phase column chromatography (ethyl acetate: petroleum ether = 0% to 50%) to afford compound **3-4** (185.9 mg, yield: 92.7%) as a yellow solid. MS (ESI, m/z): 331.8 [M+H]⁺.

To a solution of compound **3-4** (186 mg, 0.56 mmol) in tetrahydrofuran (2 mL) and dichloromethane (2 mL), phosphorus tribromide (167 mg, 0.62 mmol) was added dropwise. The reaction solution was stirred at room temperature for 2 hours. The reaction solution was evaporated to dryness via rotary evaporation, and purified by normal phase column chromatography (ethyl acetate: petroleum ether = 0% to 50%) to afford compound **3-5** (184.2 mg, yield: 82.2%) as a yellow solid. MS (ESI, m/z): 395.8 [M+H]⁺.

A mixture of compound **3-5** (184 mg, 0.456 mmol), compound **1-1** (101 mg, 0.698 mmol), potassium carbonate (128 mg, 0.93 mmol), DMF (3 mL), and water (1 mL) was stirred at 100°C for 1 hour. Upon completion of the reaction, the reaction solution was directly concentrated, and then purified by reverse-phase column chromatography [acetonitrile/water (containing 0.05% aqueous ammonia) = 0% to 100%] to afford compound 3 (100.1 mg, yield: 46.3%) as a white solid. MS (ESI, m/z): 460.8 [M+H]⁺.

¹H NMR (400 MHz, DMSO-*d*6) δ 8.27 - 8.17 (m, 1H), 8.06 - 7.96 (m, 1H), 7.93 - 7.86 (m, 2H), 7.83 - 7.76 (m, 1H), 7.76 - 7.70 (m, 1H), 7.49 - 7.39 (m, 2H), 6.11 - 6.00 (m, 1H), 5.63 (s, 2H).

### Example 4: Synthesis of Compound 4

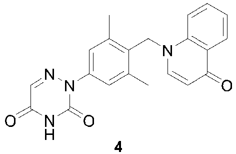

### Synthetic route:

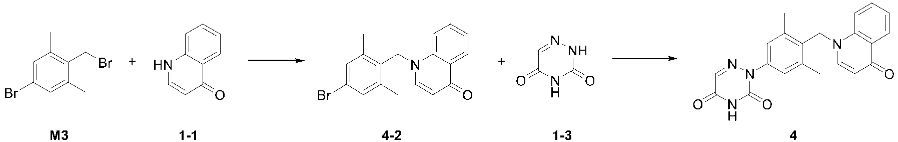

Intermediate **M3** was used as a starting material and the synthetic route of compound **1** was followed to synthesize compound **4,** which was then purified by reverse-phase column chromatography [acetonitrile/water (containing 0.05% aqueous ammonia) = 0% to 100%] to afford a white solid (20.2 mg, yield: 18.2%). MS (ESI, m/z): 374.8 [M+H]⁺.

¹H NMR (400 MHz, DMSO-d6) δ 8.24 (d, *J* = 8.0 Hz, 1H), 8.02 (d, *J* = 8.4 Hz, 1H), 7.81 (t, *J= 8.0* Hz, 1H), 7.46 (t, *J* = 7.6 Hz, 1H), 7.34 (s, 2H), 7.28 - 7.20 (m, 2H), 6.03 (d, *J =* 7.6 Hz, 1H), 5.40 (s, 2H), 2.23 (s, 6H).

### Example 5: Synthesis of Compound 5

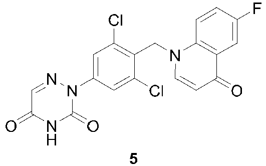

### Synthetic route:

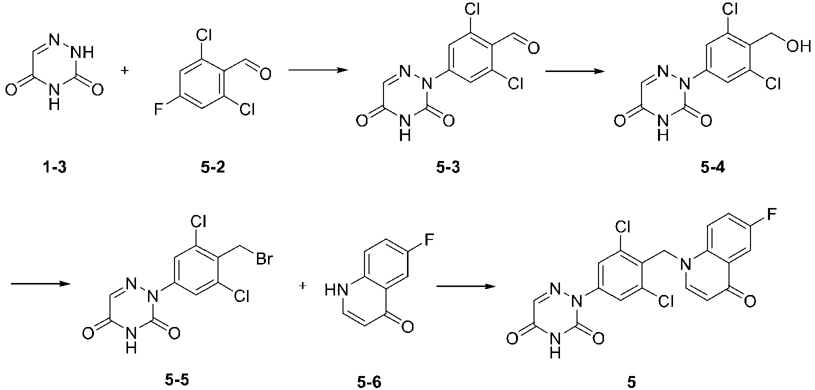

With the exception of replacing compound **3-2** with compound **5-2** and replacing compound **1-1** with compound **5-6,** the synthetic route of compound **3** was followed to synthesize compound **5,** which was then purified by reverse-phase column chromatography [acetonitrile/water (containing 0.05% aqueous ammonia) = 0% to 100%] to afford a white solid (21.8 mg, yield: 17.6%). MS (ESI, m/z): 432.8[M+H]⁺.

¹H NMR (400 MHz, DMSO-d6) δ 12.54 (s, 1H), 7.98 - 7.91 (m, 1H), 7.89 - 7.81 (m, 3H), 7.76 - 7.68 (m, 2H), 7.58 (d, *J =* 8.0 Hz, 1H), 6.08 (d, *J =* 8.0 Hz, 1H), 5.68 (s, 2H).

### Example 6: Synthesis of Compound 6

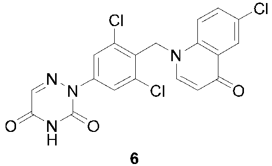

### Synthetic route:

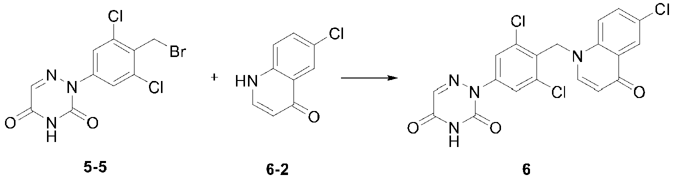

With the exception of replacing compound **5-6** with compound **6-2,** the synthetic route of compound **5** was followed to synthesize compound **6,** which was then purified by reverse-phase column chromatography [acetonitrile/water (containing 0.05% aqueous ammonia) = 0% to 100%] to afford a white solid (38.1 mg, yield: 29.7%). MS (ESI, m/z): 448.8[M+H]⁺.

¹H NMR (400 MHz, DMSO-d6) δ 12.52 (s, 1H), 8.14 (s, 1H), 7.91 (d, *J* = 8.8 Hz, 1H), 7.86 - 7.82 (m, 3H), 7.74 (s, 1H), 7.59 (d, *J* = 8.0 Hz, 1H), 6.12 (d, *J* = 8.0 Hz, 1H), 5.67 (s, 2H).

### Example 7: Synthesis of Compound 7

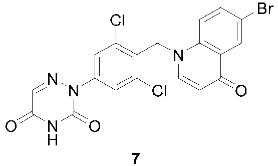

### Synthetic route:

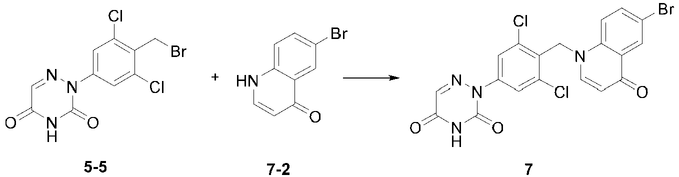

With the exception of replacing compound **5-6** with compound **7-2,** the synthetic route of compound **5** was followed to synthesize compound **7,** which was then purified by reverse-phase column chromatography [acetonitrile/water (containing 0.05% aqueous ammonia) = 0% to 100%] to afford a white solid (24.6 mg, yield: 17.4%). MS (ESI, m/z): 492.8[M+H]⁺.

¹H NMR (400 MHz, DMSO-d6) δ 12.52 (s, 1H), 8.28 (d, *J* = 2.0 Hz, 1H), 7.98 - 7.92 (m, 1H), 7.85 (s, 1H), 7.83 (s, 2H), 7.74 (s, 1H), 7.59 (d, *J* = 8.0 Hz, 1H), 6.12 (d, *J* = 8.0 Hz, 1H), 5.66 (s, 2H).

### Example 8: Synthesis of Compound 8

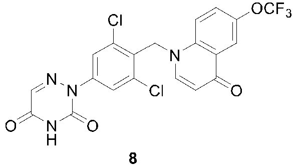

### Synthetic route:

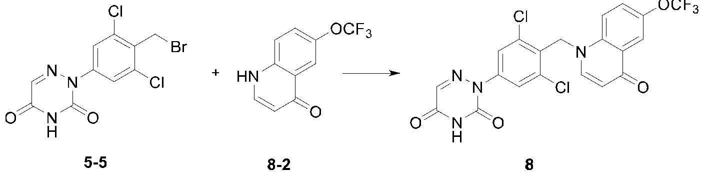

With the exception of replacing compound **5-6** with compound **8-2,** the synthetic route of compound **5** was followed to synthesize compound **8,** which was then purified by reverse-phase column chromatography [acetonitrile/water (containing 0.05% aqueous ammonia) = 0% to 100%] to afford a white solid (14.2 mg, yield: 9.9%). MS (ESI, m/z): 498.8[M+H]⁺.

¹H NMR (400 MHz, DMSO-d6) δ 12.53 (s, 1H), 8.05 (s, 1H), 8.02 (d, *J =* 9.2 Hz, 1H), 7.87 - 7.81 (m, 3H), 7.74 (s, 1H), 7.63 (d, *J =* 8.0 Hz, 1H), 6.14 (d, *J =* 8.0 Hz, 1H), 5.70 (s, 2H).

### Example 9: Synthesis of Compound 9

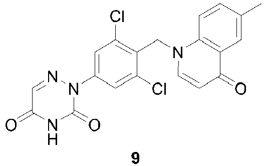

### Synthetic route:

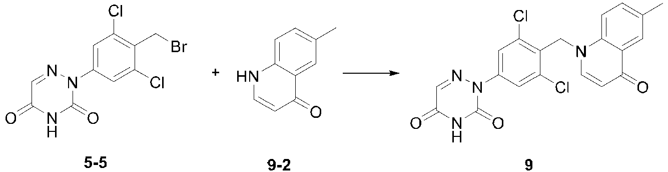

With the exception of replacing compound **5-6** with compound **9-2,** the synthetic route of compound **5** was followed to synthesize compound **9,** which was then purified by reverse-phase column chromatography [acetonitrile/water (containing 0.05% aqueous ammonia) = 0% to 100%] to afford a white solid (11.2 mg, yield: 9.1%). MS (ESI, m/z): 428.8[M+H]⁺.

¹H NMR (400 MHz, DMSO-d6) δ 8.00 (s, 1H), 7.82 (s, 2H), 7.76 - 7.70 (m, 2H), 7.59 (d, *J* = 8.8 Hz, 1H), 7.52 (d, *J =* 8.0 Hz, 1H), 6.03 (d, *J =* 8.0 Hz, 1H), 5.63 (s, 2H), 2.42 (s, 3H).

### Example 10: Synthesis of Compound 10

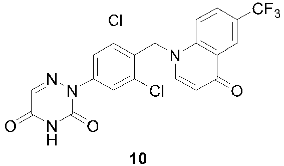

### Synthetic route:

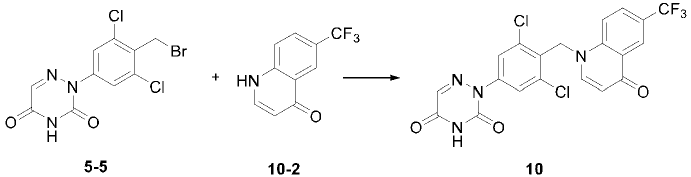

With the exception of replacing compound **5-6** with compound **10-2,** the synthetic route of compound **5** was followed to synthesize compound **10,** which was then purified by reverse-phase column chromatography [acetonitrile/water (containing 0.05% aqueous ammonia) = 0% to 100%] to afford a white solid (15.3 mg, yield: 11.1%). MS (ESI, m/z): 482.8[M+H]⁺.

¹H NMR (400 MHz, DMSO-d6) δ 12.54 (s, 1H), 8.46 (s, 1H), 8.15 - 8.04 (m, 2H), 7.83 (s, 2H), 7.74 (s, 1H), 7.68 (d, *J* = 8.0 Hz, 1H), 6.20 (d, *J* = 8.0 Hz, 1H), 5.73 (s, 2H).

### Example 11: Synthesis of Compound 11

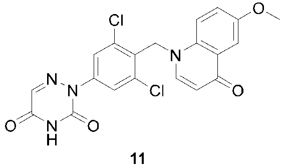

### Synthetic route:

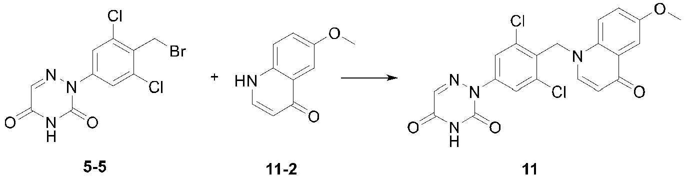

With the exception of replacing compound **5-6** with compound **11-2,** the synthetic route of compound **5** was followed to synthesize compound **11,** which was then purified by reverse-phase column chromatography [acetonitrile/water (containing 0.05% aqueous ammonia) = 0% to 100%] to afford a white solid (32.9 mg, yield: 25.9%). MS (ESI, m/z): 444.8[M+H]⁺.

¹H NMR (400 MHz, DMSO-*d*6) δ 12.53 (s, 1H), 7.85 - 7.78 (m, 3H), 7.74 (s, 1H), 7.63 (d, *J =* 2.8 Hz, 1H), 7.49 (d, *J* = 8.0 Hz, 1H), 7.44 - 7.37 (m, 1H), 6.03 (d, *J =* 8.0 Hz, 1H), 5.64 (s, 2H), 3.85 (s, 3H).

### Example 12: Synthesis of Compound 12

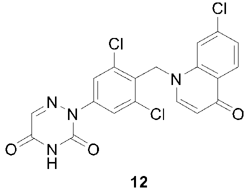

### Synthetic route:

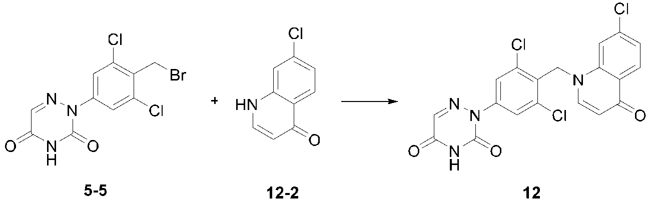

With the exception of replacing compound **5-6** with compound **12-2,** the synthetic route of compound **5** was followed to synthesize compound **12,** which was then purified by reverse-phase column chromatography [acetonitrile/water (containing 0.05% aqueous ammonia) = 0% to 100%] to afford a white solid (28.3 mg, yield: 21.9%). MS (ESI, m/z): 448.8[M+H]⁺.

¹H NMR (400 MHz, DMSO-*d*6) δ 8.20 (d, *J* = 8.8 Hz, 1H), 8.00 (s, 1H), 7.84 (s, 2H), 7.73 (s, 1H), 7.55 (d, *J =* 8.0 Hz, 1H), 7.47 (d, *J* = 8.8 Hz, 1H), 6.09 (d, *J* = 8.0 Hz, 1H), 5.64 (s, 2H).

### Example 13: Synthesis of Compound 13

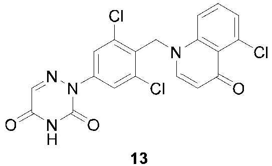

### Synthetic route:

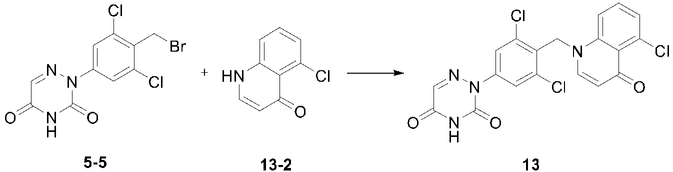

With the exception of replacing compound **5-6** with compound **13-2,** the synthetic route of compound **5** was followed to synthesize compound **13,** which was then purified by reverse-phase column chromatography [acetonitrile/water (containing 0.05% aqueous ammonia) = 0% to 100%] to afford a white solid (10.2 mg, yield: 16.2%). MS (ESI, m/z): 448.8[M+H]⁺.

¹H NMR (400 MHz, DMSO-d6) δ 7.81 (s, 2H), 7.76 - 7.69 (m, 2H), 7.64 (t, *J =* 7.2 Hz, 1H), 7.49 (d, *J =* 8.0 Hz, 1H), 7.38 (d, *J =* 6.0 Hz, 1H), 6.04 (d, *J =* 8.0Hz, 1H), 5.59 (s, 2H).

### Example 14: Synthesis of Compound 14

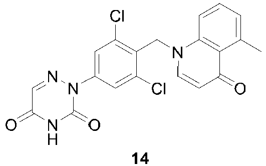

### Synthetic route:

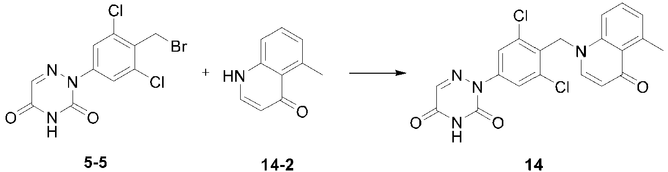

With the exception of replacing compound **5-6** with compound **14-2,** the synthetic route of compound **5** was followed to synthesize compound **14,** which was then purified by reverse-phase column chromatography [acetonitrile/water (containing 0.05% aqueous ammonia) = 0% to 100%] to afford a white solid (6.6 mg, yield: 5.4%). MS (ESI, m/z): 428.8[M+H]⁺.

¹H NMR (400 MHz, DMSO-*d*6) δ 7.82 (s, 2H), 7.73 (s, 1H), 7.60 - 7.55 (m, 2H), 7.44 (d, *J* = 8.0 Hz, 1H), 7.12 (d, *J* = 5.6 Hz, 1H), 5.98 (d, *J* = 8.0 Hz, 1H), 5.56 (s, 2H), 2.82 (s, 3H).

### Example 15: Synthesis of Compound 17

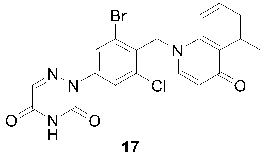

### Synthetic route:

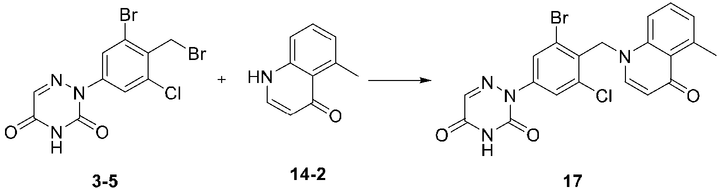

With the exception of replacing compound **1-1** with compound **14-2,** the synthetic route of compound 3 was followed to synthesize compound **17,** which was then purified by reverse-phase column chromatography [acetonitrile/water (containing 0.05% aqueous ammonia) = 0% to 100%] to afford a white solid (27.8 mg, yield: 28.1%). MS (ESI, m/z): 472.8[M+H]⁺.

¹H NMR (400 MHz, DMSO-d6) δ 12.52 (s, 1H), 7.99 (s, 1H), 7.86 (s, 1H), 7.73 (s, 1H), 7.67 - 7.57 (m, 2H), 7.30 (d, *J=* 7.6 Hz, 1H), 7.14 (d, *J=* 7.2 Hz, 1H), 5.97 (d, *J=* 8.0 Hz, 1H), 5.53 (s, 2H), 2.83 (s, 3H).

### Example 16: Synthesis of Compound 18

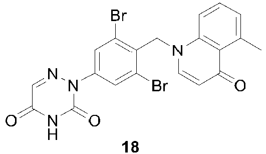

### Synthetic route:

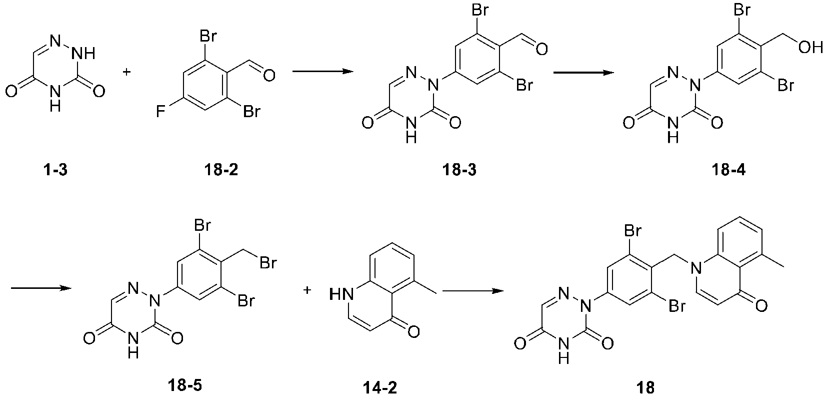

With the exception of replacing compound **3-2** with compound **18-2** and replacing compound **1-1** with compound **14-2,** the synthetic route of compound **3** was followed to synthesize compound **18,** which was then purified by reverse-phase column chromatography [acetonitrile/water (containing 0.05% aqueous ammonia) = 0% to 100%] to afford a white solid (13.5 mg, yield: 11.4%). MS (ESI, m/z): 518.8[M+H]⁺.

¹H NMR (400 MHz, DMSO-*d*6) δ 12.50 (s, 1H), 8.02 (s, 2H), 7.77 - 7.69 (m, 2H), 7.64 - 7.56 (m, 1H), 7.20 - 7.12 (m, 2H), 5.96 (d, *J* = 8.0 Hz, 1H), 5.50 (s, 2H), 2.83 (s, 3H).

### Example 17: Synthesis of Compound 19

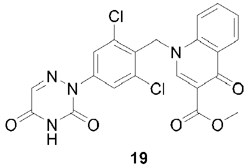

### Synthetic route:

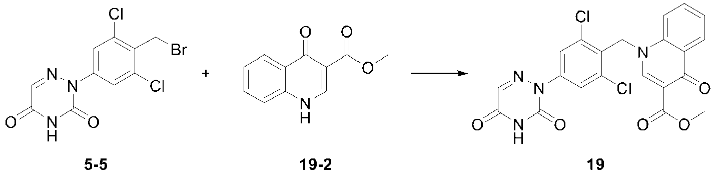

With the exception of replacing compound **5-6** with compound **19-2,** the synthetic route of compound **5** was followed to synthesize compound **19,** which was then purified by reverse-phase column chromatography [acetonitrile/water (containing 0.05% aqueous ammonia) = 0% to 100%] to afford a white solid (560.5 mg, yield: 82.6%). MS (ESI, m/z): 472.8[M+H]⁺.

¹H NMR (400 MHz, DMSO-d6) δ 12.53 (s, 1H), 8.40 (s, 1H), 8.29 (d, *J* = 8.0 Hz, 1H), 7.85 (s, 2H), 7.83 - 7.78 (m, 2H), 7.73 (s, 1H), 7.55 - 7.48 (m, 1H), 5.82 (s, 2H), 3.72 (s, 3H).

### Example 18: Synthesis of Compound 20

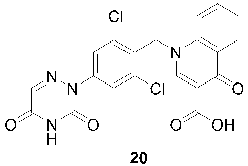

### Synthetic route:

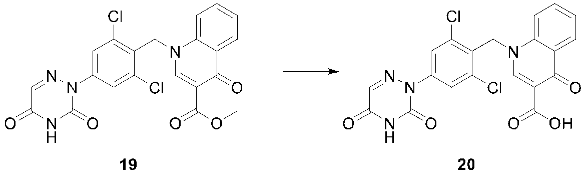

A mixture of compound **19** (57.6 mg, 0.122 mmol), lithium hydroxide (147 mg, 6.1 mmol), methanol (2 mL), water (1 mL), and tetrahydrofuran (2 mL) was stirred at 60°C for 3 hours. Upon completion of the reaction, the reaction solution was directly concentrated, and then purified by reverse-phase column chromatography [acetonitrile/water (containing 0.05% aqueous ammonia) = 0% to 100%] to afford compound **20** (23.6 mg, yield: 42.3%) as a yellow solid. MS (ESI, m/z): 458.8 [M+H]⁺.

¹H NMR (400 MHz, DMSO-*d*6) δ 14.95 (s, 1H), 12.53 (s, 1H), 8.63 (s, 1H), 8.45 (d, *J* = 8.0 Hz, 1H), 8.08 - 8.00 (m, 2H), 7.88 (s, 2H), 7.78 - 7.68 (m, 2H), 6.03 (s, 2H).

### Example 19: Synthesis of Compound 21

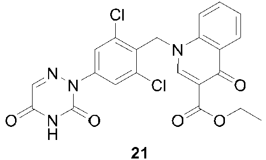

### Synthetic route:

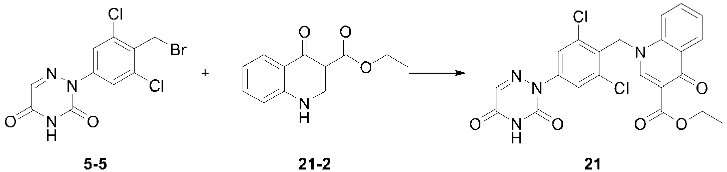

With the exception of replacing compound **5-6** with compound **21-2,** the synthetic route of compound **5** was followed to synthesize compound **21,** which was then purified by reverse-phase column chromatography [acetonitrile/water (containing 0.05% aqueous ammonia) = 0% to 100%] to afford a white solid (15.3 mg, yield: 11.1%). MS (ESI, m/z): 486.8[M+H]⁺.

¹H NMR (400 MHz, DMSO-*d*6) δ 12.54 (s, 1H), 8.32 (s, 1H), 8.30 (d, *J =* 8.0 Hz, 1H), 7.87 - 7.82 (m, 3H), 7.82 - 7.77 (m, 1H), 7.70 (s, 1H), 7.54 - 7.46 (m, 1H), 5.78 (s, 2H), 4.20 - 4.13 (m, 2H), 1.25 - 1.21 (m, 3H).

### Example 20: Synthesis of Compound 22

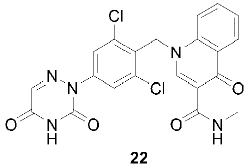

### Synthetic route:

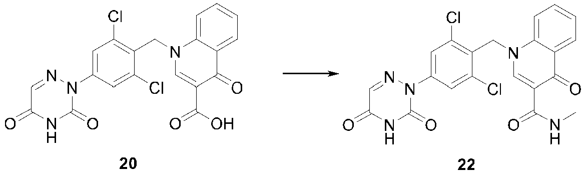

A mixture of compound **20** (25.2 mg, 0.055 mmol), oxalyl chloride (0.5 mL), N,N-dimethylformamide (0.1 mL), and dichloromethane (5 mL) was stirred at room temperature and reacted for 10 min. After the solvent was removed under vacuum from the reaction solution, N,N-dimethylformamide (5 mL), methylamine hydrochloride (11 mg, 0.164 mmol), and triethylamine (1 mL) were added and stirred at room temperature for 3 hours. Upon completion of the reaction, the reaction solution was directly concentrated, and then purified by reverse-phase column chromatography [acetonitrile/water (containing 0.05% aqueous ammonia) = 0% to 100%] to afford compound **22** (1.6 mg, yield: 6.1%) as a white solid. MS (ESI, m/z): 472.3 [M+H]⁺.

¹H NMR (400 MHz, DMSO-d6) δ 12.53 (s, 1H), 9.71 - 9.63 (m, 1H), 8.46 (s, 1H), 8.39 (d, *J* = 8.0 Hz, 1H), 8.00 (d, *J* = 8.8 Hz, 1H), 7.91 (d, *J* = 7.2 Hz, 1H), 7.88 (s, 2H), 7.73 (s, 1H), 7.59 (t, *J* = 7.2 Hz, 1H), 5.87 (s, 2H), 2.81 (d, *J* = 3.6 Hz, 3H).

### Example 21: Synthesis of Compound 23

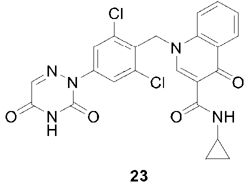

### Synthetic route:

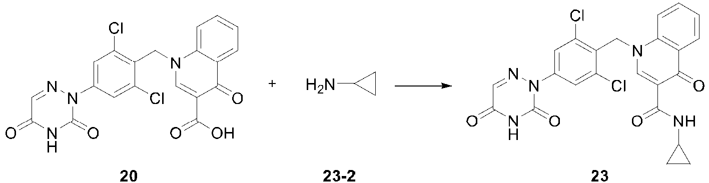

A mixture of compound **20** (25.2 mg, 0.055 mmol), oxalyl chloride (0.5 mL), N,N-dimethylformamide (0.1 mL), and dichloromethane (5 mL) was reacted with stirring at room temperature for 10 min. After the solvent was removed under vacuum from the reaction solution, N,N-dimethylformamide (5 mL) and cyclopropylamine **23-2** (0.5 mL) were added and stirred at room temperature for 3 hours. Upon completion of the reaction, the reaction solution was directly concentrated, and then purified by reverse-phase column chromatography [acetonitrile/water (containing 0.05% aqueous ammonia) = 0% to 100%] to afford compound **23** (0.9 mg, yield: 3.1%) as a white solid. MS (ESI, m/z): 498.3 [M+H]⁺.

¹H NMR (400 MHz, DMSO-*d*6) δ 9.87 - 9.82 (m, 1H), 8.45 (s, 1H), 8.37 (d, *J* = 7.6 Hz, 1H), 8.02 (d, *J* = 8.8 Hz, 1H), 7.96 - 7.90 (m, 1H), 7.90 (s, 2H), 7.69 (s, 1H), 7.61 - 7.58 (m, 1H), 5.87 (s, 2H), 2.85 - 2.77 (m, 1H), 0.72 - 0.70 (m, 2H), 0.52 - 0.51 (m, 2H).

### Example 22: Synthesis of Compound 24

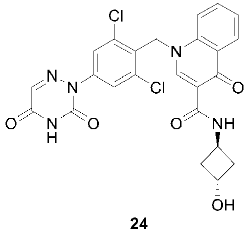

### Synthetic route:

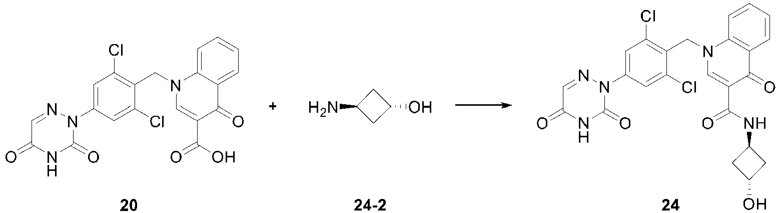

A mixture of compound **20** (20.2 mg, 0.044 mmol), compound **24-2** (5.4 mg, 0.044 mmol), 2-(7-azabenzotriazol-1-yl)-N,N,N',N'-tetramethyluronium hexafluorophosphate (33.5 mg, 0.088 mmol), N,N-diisopropylethylamine (22.7 mg, 0.176 mmol), and N,N-dimethylformamide (2 mL) was reacted with stirring overnight at room temperature. Upon completion of the reaction, the reaction solution was directly concentrated, and then purified by reverse-phase column chromatography [acetonitrile/water (containing 0.05% aqueous ammonia) = 0% to 100%] to afford compound **24** (5.1 mg, yield: 21.7%) as a yellow solid. MS (ESI, m/z): 528.3 [M+H]⁺.

¹H NMR (400 MHz, DMSO-d6) δ 10.05 (d, *J* = 6.8 Hz, 1H), 8.43 - 8.35 (m, 2H), 8.03 (d, *J = 8.8* Hz, 1H), 7.94 - 7.85 (m, 3H), 7.63 - 7.54 (m, 2H), 5.84 (s, 2H), 4.40 - 4.32 (m, 1H), 4.32 - 4.25 (m, 1H), 2.17 (t, *J =* 6.0 Hz, 4H).

### Example 23: Synthesis of Compound 25

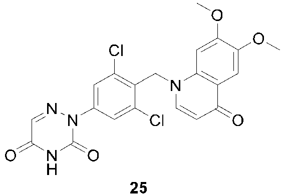

### Synthetic route:

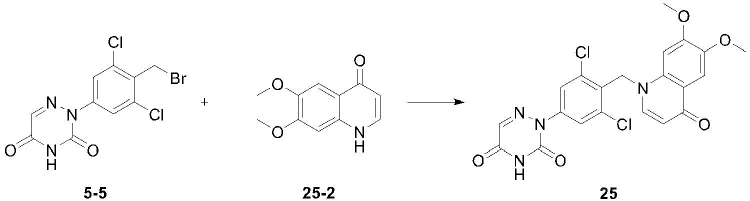

With the exception of replacing compound **5-6** with compound **25-2,** the synthetic route of compound **5** was followed to synthesize compound **25,** which was then purified by reverse-phase column chromatography [acetonitrile/water (containing 0.05% aqueous ammonia) = 0% to 100%] to afford a white solid (28.1 mg, yield: 20.6%). MS (ESI, m/z): 474.8[M+H]⁺.

¹H NMR (400 MHz, DMSO-*d*6) δ 12.54 (s, 1H), 7.83 (s, 2H), 7.73 (s, 1H), 7.57 (s, 1H), 7.50 (d, *J* = 8.0 Hz, 1H), 7.11 (s, 1H), 5.99 (d, *J* = 7.6 Hz, 1H), 5.67 (s, 2H), 3.90 (s, 3H), 3.84 (s, 3H).

### Example 24: Synthesis of Compound 26

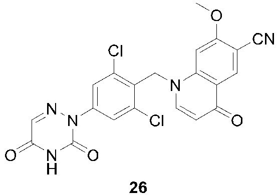

### Synthetic route:

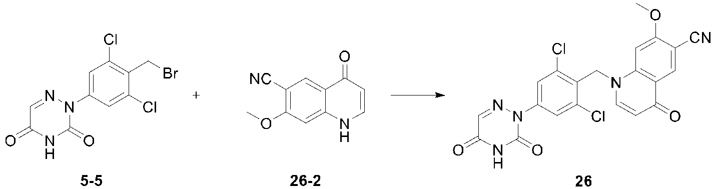

With the exception of replacing compound **5-6** with compound **26-2,** the synthetic route of compound **5** was followed to synthesize compound **26,** which was then purified by reverse-phase column chromatography [acetonitrile/water (containing 0.05% aqueous ammonia) = 0% to 100%] to afford a white solid (20.1 mg, yield: 14.7%). MS (ESI, m/z): 469.8[M+H]⁺.

¹H NMR (400 MHz, DMSO-*d*6) δ 8.42 (s, 1H), 7.86 (s, 2H), 7.71 (s, 1H), 7.59 (d, *J* = 8.0 Hz, 1H), 7.29 (s, 1H), 6.10 (d, *J =* 8.0 Hz, 1H), 5.70 (s, 2H), 4.04 (s, 3H).

### Example 25: Synthesis of Compound 27

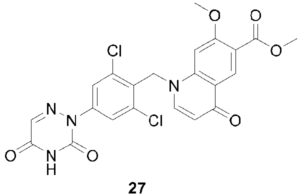

### Synthetic route:

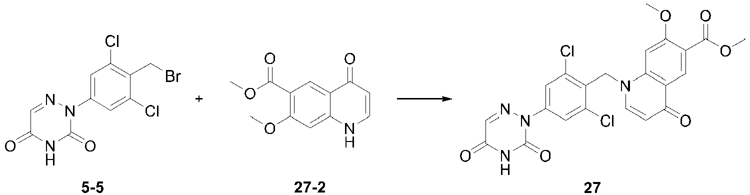

With the exception of replacing compound **5-6** with compound **27-2,** the synthetic route of compound **5** was followed to synthesize compound **27,** which was then purified by reverse-phase column chromatography [acetonitrile/water (containing 0.05% aqueous ammonia) = 0% to 100%] to afford a white solid (57.5 mg, yield: 39.9%). MS (ESI, m/z): 502.8[M+H]⁺.

¹H NMR (400 MHz, DMSO-*d*6) δ 8.52 (s, 1H), 7.84 (s, 2H), 7.70 (s, 1H), 7.60 (d, *J* = 8.0 Hz, 1H), 7.12 (s, 1H), 6.06 (d, *J=* 8.0 Hz, 1H), 5.69 (s, 2H), 3.93 (s, 3H), 3.82 (s, 3H).

### Example 26: Synthesis of Compound 28

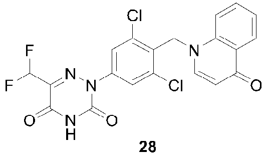

### Synthetic route:

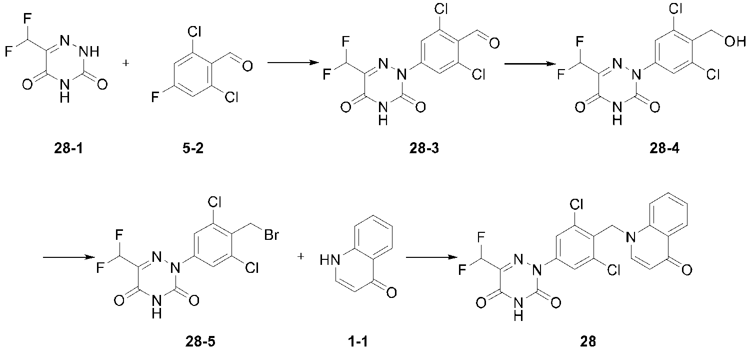

With the exception of replacing compound **1-3** with compound **28-1** and replacing compound **3-2** with compound **5-2,** the synthetic route of compound **3** was followed to synthesize compound **28,** which was then purified by reverse-phase column chromatography [acetonitrile/water (containing 0.05% aqueous ammonia) = 0% to 100%] to afford a white solid (46.3 mg, yield: 42.2%). MS (ESI, m/z): 464.8 [M+H]⁺.

¹H NMR (400 MHz, DMSO-*d*6) δ 12.88 (s, 1H), 8.21 (d, *J* = 8.0 Hz, 1H), 7.89 - 7.70 (m, 4H), 7.57 (d*, J* = 8.0 Hz, 1H), 7.42 (t, *J* = 7.6 Hz, 1H), 6.91 (t, *J* = 52.0 Hz, 1H), 6.07 (d, *J* = 8.0 Hz, 1H), 5.66 (s, 2H).

### Example 27: Synthesis of Compound 29

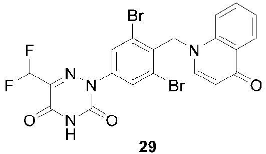

### Synthetic route:

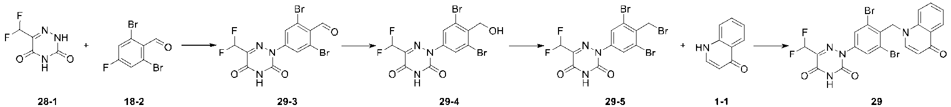

With the exception of replacing compound **5-2** with compound **18-2,** the synthetic route of compound **28** was followed to synthesize compound **29,** which was then purified by reverse-phase column chromatography [acetonitrile/water (containing 0.05% aqueous ammonia) = 0% to 100%] to afford a white solid (5.1 mg, yield: 2.2%). MS (ESI, m/z): 552.8 [M+H]⁺.

¹H NMR (400 MHz, DMSO-*d*6) δ 8.22 (d, *J =* 7.6 Hz, 1H), 8.03 (s, 2H), 7.96 (d, *J* = 8.8 Hz, 1H), 7.81 (t, *J* = 8.0 Hz, 1H), 7.45 (t, *J =* 8.0 Hz, 1H), 7.33 (d, *J =* 8.0 Hz, 1H), 6.90 (t, *J* = 52.8 Hz, 1H), 6.05 (d, *J* = 7.6 Hz, 1H), 5.61 (s, 2H).

### Example 28: Synthesis of Compound 30

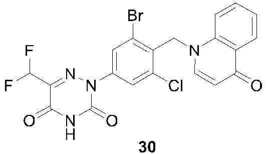

### Synthetic route:

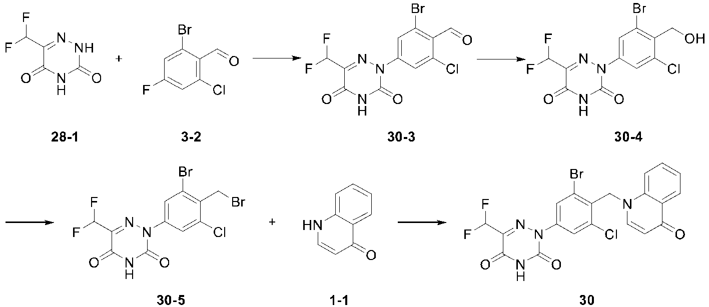

With the exception of replacing compound **1-3** with compound **28-1,** the synthetic route of compound **3** was followed to synthesize compound **30,** which was then purified by reverse-phase column chromatography [acetonitrile/water (containing 0.05% aqueous ammonia) = 0% to 100%] to afford a white solid (30.3 mg, yield: 49.1%). MS (ESI, m/z): 508.8[M+H]⁺.

¹H NMR (400 MHz, DMSO-*d*6) δ 8.23 (d, *J* = 8.0 Hz, 1H), 7.99 (s, 1H), 7.94 - 7.85 (m, 2H), 7.80 (t, *J =* 8.0 Hz, 1H), 7.51 - 7.39 (m, 2H), 6.93 (t, *J =* 52.4 Hz, 1H), 6.07 (d, *J =* 8.0 Hz, 1H), 5.64 (s, 2H).

### Example 29: Synthesis of Compound 31

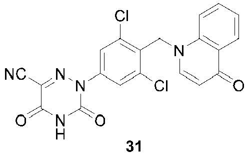

### Synthetic route:

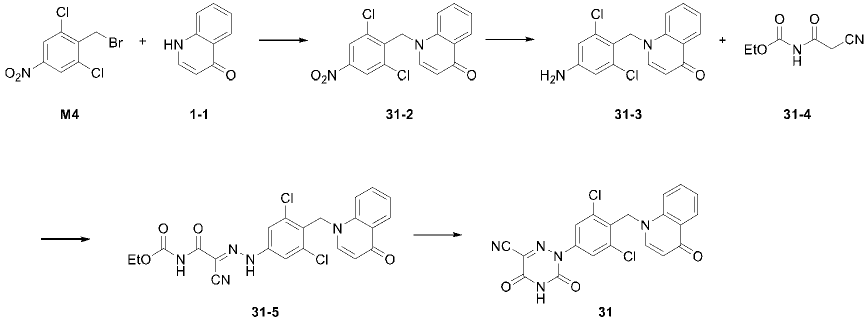

The synthetic method for intermediate **M1** was followed to afford intermediate **M4,** and subsequently the synthetic route of compound **1** was followed to synthesize compound **31-2,** which was then purified by reverse-phase column chromatography [acetonitrile/water (containing 0.05% aqueous ammonia) = 0% to 100%] to afford a yellow solid (479 mg, 1.38 mmol). Thereafter, compound **31-2** was reacted overnight at 60°C with a mixture of sodium dithionite (960 mg, 5.52 mmol), THF (8 mL), and water (4 mL). Upon completion of the reaction, the reaction solution was directly concentrated, and then purified by normal phase column chromatography (ethyl acetate: petroleum ether = 0% to 50%) to afford compound **31-3** (300.2 mg, yield: 68.2%) as a yellow solid. MS (ESI, m/z): 318.8 [M+H]⁺.

A mixture of compound **31-3** (130 mg, 0.41 mmol), sodium nitrite (34 mg, 0.49 mmol, 0.5 mL aqueous solution), concentrated hydrochloric acid (0.07 mL), and acetic acid (3 mL) was stirred at 0°C for 0.5 hour. Compound **31-4** (192 mg, 1.23 mmol) was added and further stirred for 2 hours. Upon completion of the reaction, the reaction solution was directly concentrated, and then purified by normal phase column chromatography (ethyl acetate: petroleum ether = 0% to 50%) to afford compound **31-5** (56.8 mg, yield: 29.1%) as a white solid. MS (ESI, m/z): 485.8 [M+H]⁺.

A mixture of compound **31-5** (57 mg, 0.118 mmol), potassium acetate (23 mg, 0.236 mmol), and acetic acid (3 mL) was stirred at 115°C for 4 hours. Upon completion of the reaction, the reaction solution was directly concentrated, and then purified by reverse-phase column chromatography [acetonitrile/water (containing 0.05% aqueous ammonia) = 0% to 100%] to afford compound **31** (20.1 mg, yield: 39.0%) as a white solid. MS (ESI, m/z): 439.8 [M+H]⁺.

¹H NMR (400 MHz, DMSO-d6) δ 8.21 (d, *J* = 8.0 Hz, 1H), 7.86 - 7.72 (m, 4H), 7.57 (d, *J* = 8.0 Hz, 1H), 7.42 (t, *J* = 7.2 Hz, 1H), 6.06 (d, *J =* 8.0 Hz, 1H), 5.66 (s, 2H).

### Example 30: Synthesis of Compound 32

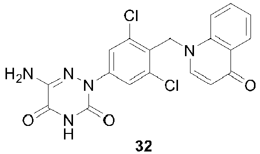

### Synthetic route:

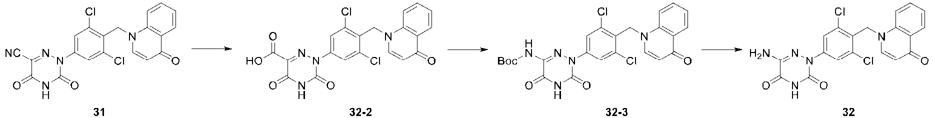

Compound **31** (150 mg, 0.34 mmol) and concentrated hydrochloric acid (1 mL) were added to an acetic acid (5 mL) solution, and then heated to 120°C, and reacted overnight. Upon completion of the reaction, the reaction solution was directly concentrated, and then purified by normal phase column chromatography (ethyl acetate: petroleum ether = 0% to 50%) to afford a yellow oily product **32-2** (139.8 mg, yield: 89.7%). MS(ESI, m/z): 458.8 [M+H]⁺.

Compound **32-2** (95 mg, 0.21 mmol), diphenylphosphoryl azide (177 mg, 0.64 mmol), and trimethylamine (65 mg, 0.64 mmol) were added to a mixed solution of tetrahydrofuran (3 mL) and tertiary butanol (10 mL), and then heated to 85°C, and reacted overnight. Upon completion of the reaction, the reaction solution was directly concentrated, and then purified by normal phase column chromatography (ethyl acetate: petroleum ether = 0% to 50%) to afford compound **32-3** (40.2 mg, yield: 36.4%) as a yellow solid. MS(ESI, m/z): 529.8 [M+H]⁺.

Compound **32-3** (40 mg, 0.075 mmol) was added to a mixed solution of tetrahydrofuran (5 mL) and dioxane (20 mL). Thereafter, under the ice-bath condition, trifluoroacetic acid (5 mL) was added to the reaction solution and reacted at room temperature for 2 hours. Upon completion of the reaction, the reaction solution was directly concentrated, and then purified by reverse-phase column chromatography [acetonitrile/water (containing 0.05% aqueous ammonia) = 0% to 100%] to afford compound **32** (9.8 mg, yield: 30.2%) as a white solid. MS(ESI, m/z): 429.8 [M+H]⁺.

¹HNMR (400 MHz, DMSO-*d*6) δ 12.32 (s, 1H), 8.22 (d, *J* = 8.0 Hz, 1H), 7.96 (s, 2H), 7.84 (d, *J* = 8.0 Hz, 1H), 7.78 (t, *J* = 7.6 Hz, 1H), 7.53 (d, *J* = 7.6 Hz, 1H), 7.43 (t, *J =* 7.2 Hz, 1H), 6.59 (s, 2H), 6.08 (d, *J* = 7.6 Hz, 1H), 5.63 (s, 2H).

### Example 31: Synthesis of Compound 33

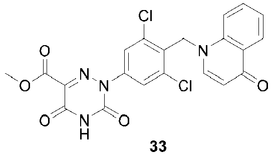

### Synthetic route:

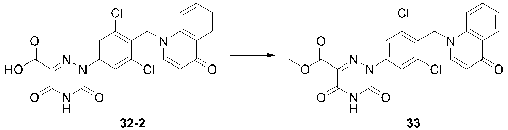

The synthetic route of compound **32** was followed to afford compound **32-2** (1.6 g, 3.49 mmol), and compound **32-2** was added to a methanol (20 mL) solution. Thereafter, under the ice-bath condition, sulfoxide chloride (3 mL) was slowly added to the reaction solution, heated to 80°C, and reacted for 2 hours. Upon completion of the reaction, the reaction solution was directly concentrated, and then purified by reverse-phase column chromatography [acetonitrile/water (containing 0.05% aqueous ammonia) = 0% to 100%] to afford compound **33** (619.8 mg, yield: 37.6%) as a white solid. MS(ESI, m/z): 472.8[M+H]⁺.

¹H NMR (400 MHz, DMSO-d6) δ 12.81 (s, 1H), 8.22 (d, *J* = 8.0 Hz, 1H), 7.84 - 7.76 (m, 4H), 7.58 (d, *J =* 8.0 Hz, 1H), 7.43 (t, *J =* 7.6 Hz, 1H), 6.07 (d, *J =* 7.6 Hz, 1H), 5.67 (s, 2H), 3.85 (s, 3H).

### Example 32: Synthesis of Compound 34

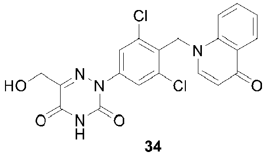

### Synthetic route:

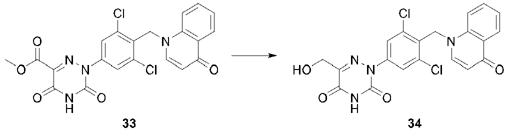

Compound **33** (620 mg, 1.31 mmol) was added to a mixed solution of tetrahydrofuran (10 mL) and methanol (10 mL). Thereafter, under the ice-bath condition, lithium borohydride (275 mg, 13.1 mmol) was slowly added to the reaction solution, heated to 60°C, and reacted for 6 hours. Upon completion of the reaction, the reaction solution was directly concentrated, and then purified by reverse-phase column chromatography [acetonitrile/water (containing 0.05% aqueous ammonia) = 0% to 100%] to afford compound **34** (18.8 mg, yield: 3.2%) as a yellow solid. MS(ESI, m/z): 444.8[M+H]⁺.

¹H NMR (400 MHz, DMSO-*d*6) δ 8.22 (d, *J* = 8.0 Hz, 1H), 7.93 (s, 2H), 7.85 - 7.76 (m, 2H), 7.56 (d, *J* = 8.0 Hz, 1H), 7.43 (t, *J* = 7.2 Hz, 1H), 6.08 (d, *J =* 7.6 Hz, 1H), 5.65 (s, 2H), 5.33 - 5.26 (m, 1H), 4.40 (s, 2H).

### Example 33: Synthesis of Compound 35

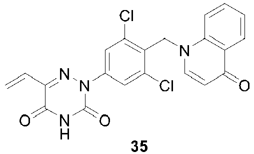

### Synthetic route:

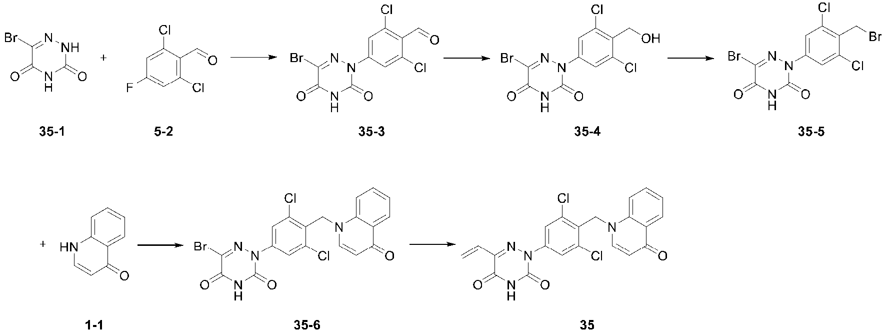

With the exception of replacing compound **28-1** with compound **35-1,** the synthesis of compound **28** was followed to synthesize compound **35-6,** which was then purified by reverse-phase column chromatography [acetonitrile/water (containing 0.05% aqueous ammonia) = 0% to 100%] to afford a white solid (415.9 mg, yield: 29.0%), MS(ESI, m/z): 492.9[M+H]⁺.

Compound **35-6** (51 mg, 0.1 mmol), potassium vinyltrifluoroborate (28 mg, 0.21 mmol), [1,1'-bis(diphenylphosphino)ferrocene]dichloropalladium (15 mg, 0.021 mmol), and potassium carbonate (43 mg, 0.31 mmol) were added to a mixed solution of N,N-dimethylformamide (4 mL) and water (1 mL), heated to 100°C, and stirred overnight. Upon completion of the reaction, the reaction solution was directly concentrated, and then purified by reverse-phase column chromatography [acetonitrile/water (containing 0.05% aqueous ammonia) = 0% to 100%] to afford compound **35** (13.6 mg, yield: 29.9%) as a gray solid, MS(ESI, m/z): 440.8[M+H]⁺.

¹H NMR (400 MHz, DMSO-d6) δ 8.21 (dd, *J =* 8.0 Hz, 1.6 Hz, 1H), 7.86 - 7.74 (m, 4H), 7.56 (d, *J =* 8.0 Hz, 1H), 7.42 (t, *J* = 7.6 Hz, 1H), 6.66 (dd, *J =* 17.6 Hz, 11.6 Hz, 1H), 6.44 (dd, *J* = 17.6 Hz, 2.0 Hz, 1H), 6.07 (d, *J =* 8.0 Hz, 1H), 5.68 - 5.63 (m, 3H).

### Example 34: Synthesis of Compound 36

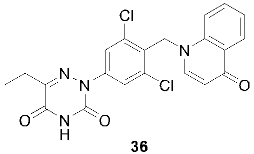

### Synthetic route:

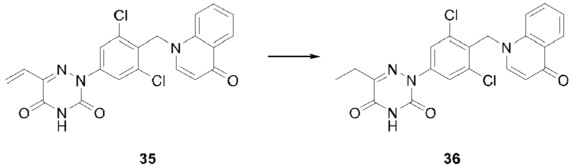

Compound **35** (6 mg, 0.014 mmol) and platinum on carbon (5 mg) were added to methanol (10 mL), and stirred at room temperature for 1 hour under hydrogen atmosphere. Upon completion of the reaction, the reaction solution was directly concentrated, and then purified by reverse-phase column chromatography [acetonitrile/water (containing 0.05% aqueous ammonia) = 0% to 100%] to afford compound **36** (1.6mg, yield: 27.3%) as a white solid, MS(ESI, m/z): 442.8[M+H]⁺.

¹H NMR (400 MHz, DMSO-*d*6) δ 8.21 (d, *J =* 8.0 Hz, 1H), 7.86 - 7.75 (m, 4H), 7.56 (d, *J* = 8.4 Hz, 1H), 7.42 (t, *J* = 7.6 Hz, 1H), 6.07 (d, *J =* 8.0 Hz, 1H), 5.64 (s, 2H), 2.58 (q, *J =* 7.2 Hz, 2H), 1.13 (t, *J* = 7.2 Hz, 3H).

### Example 35: Synthesis of Compound 37

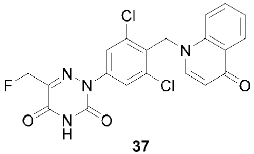

### Synthetic route:

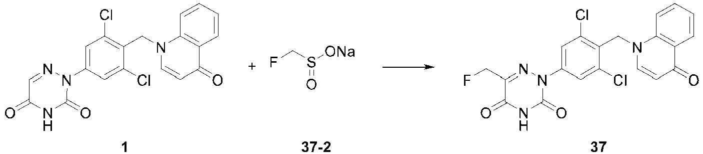

Compound **1** (107 mg, 0.26 mmol), compound **37-2** (47 mg, 0.39 mmol), and Acid Red 94 (5 mg, 0.0052 mmol) were added to dimethylsulfoxide (5 mL), and heated to 50°C and stirred for 1 hour under green light. Upon completion of the reaction, the reaction solution was directly concentrated, and then purified by reverse-phase column chromatography [acetonitrile/water (containing 0.05% aqueous ammonia) = 0% to 100%] to afford compound **37** (13.8 mg, yield: 12.0%) as a white solid, MS(ESI, m/z): 446.8[M+H]⁺.

¹H NMR (400 MHz, DMSO-d6) δ 12.70 (s, 1H), 8.22 (dd, *J* = 8.0 Hz, 1.2 Hz, 1H), 7.86 - 7.74 (m, 4H), 7.57 (d, *J* = 8.0 Hz, 1H), 7.46 - 7.40 (m, 1H), 6.07 (d, *J =* 8.0 Hz, 1H), 5.66 (s, 2H), 5.31 (d, *J* = 46.8 Hz, 2H).

### Example 36: Synthesis of Compound 38

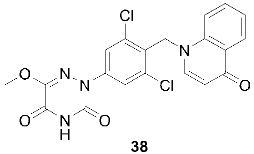

### Synthetic route:

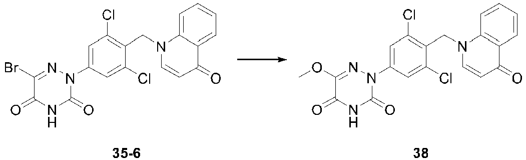

The synthetic route of compound **35** was followed to afford compound **35-6** (50 mg, 0.1 mmol), and compound **35-6** was added to a mixed solution of N,N-dimethylformamide (1.5 mL) and methanol (3 mL). Under the ice-bath condition, sodium methoxide (91 mg, 0.5 mmol) was added to the reaction solution and stirred at 85°C for 7 hours. Upon completion of the reaction, the reaction solution was directly concentrated, and then purified by reverse-phase column chromatography [acetonitrile/water (containing 0.05% aqueous ammonia) = 0% to 100%] to afford compound **38** (17.0 mg, yield: 37.8%) as a yellow solid, MS(ESI, m/z): 444.8[M+H]⁺.

¹H NMR (400 MHz, DMSO-d6) δ 12.50 (s, 1H), 8.20 (d, *J* = 7.6 Hz, 1H), 7.92 (s, 2H), 7.84 - 7.74 (m, 2H), 7.55 (d, *J* = 7.2 Hz, 1H), 7.42 (t, *J =* 7.6 Hz, 1H), 6.06 (d, *J =* 7.6 Hz, 1H), 5.63 (s, 2H), 3.85 (s, 3H).

### Example 37: Synthesis of Compound 39

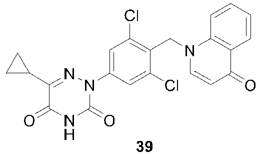

### Synthetic route:

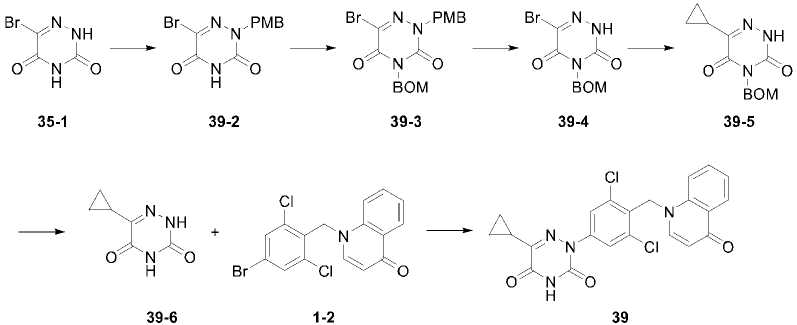

Under nitrogen atmosphere, compound **35-1** (4.9 g, 25.5 mmol) and N,O-bis(trimethylsilyl)acetamide (10.4 g, 51.0 mmol) were added to acetonitrile (0.5 mL) and stirred at 85°C for 2 hours. Afterwards, 4-methoxybenzylchloride (4.4 g, 28.1 mmol) and sodium iodide (3.83 g, 25.5 mmol) were added to the reaction solution and stirred at 85°C overnight. Upon completion of the reaction, the reaction solution was directly concentrated and purified by normal phase column chromatography (ethyl acetate: petroleum ether = 0% to 50%) to afford compound **39-2** (7.8 g, yield: 98.0%) as a yellow solid, MS(ESI, m/z): 311.8[M+H]⁺.

Compound **39-2** (7.8 g, 25 mmol), benzyl chloromethyl ether (4.3 g, 27.5 mmol), and sodium hydride (1.5 g, 37.5 mmol) were added to N,N-dimethylformamide (30 mL), and stirred at room temperature for 1 hour. Upon completion of the reaction, a saturated ammonium chloride solution (100 mL) was added to quench the system. The reaction system was extracted with ethyl acetate (200 mL). The organic phases were washed with saturated saline. The organic phases were combined and concentrated to afford a crude product. Afterwards, the crude product was purified by normal phase column chromatography (ethyl acetate: petroleum ether = 0% to 50%) to afford a yellow oily product **39-3** (10 g, yield: 92.6%), MS(ESI, m/z): 431.8[M+H]⁺.

Compound **39-3** (6.5 g, 15.0 mmol) and ceric ammonium nitrate (24.7 g, 45.1 mmol) were added to a mixed solution of acetonitrile (60 mL) and water (20 mL) and stirred overnight at room temperature. Upon completion of the reaction, the reaction solution was concentrated, then added with 200 mL of water, and subsequently extracted with ethyl acetate (200 mL). The organic phases were washed with saturated saline. The organic phases were combined and concentrated to afford a crude product. Afterwards, the crude product was purified by normal phase column chromatography (ethyl acetate: petroleum ether = 0% to 50%) to afford a yellow oily product **39-4** (2.5 g, yield: 53.3%), MS(ESI, m/z): 311.8[M+H]⁺.

Compound **39-4** (788 mg, 2.53 mmol), cyclopropylboronic acid (1 g, 12.6 mmol), and [1,1'-bis(di-tert-butylphosphino)ferrocene]dichloropalladium (326 mg, 0.51 mmol) were added to a mixed solution of 2N sodium carbonate solution (7 mL) and 1,4-dioxane (28 mL), and heated to 90°C and stirred overnight. Upon completion of the reaction, the reaction solution was extracted with ethyl acetate (100 mL). The organic phases were washed with saturated saline. The organic phases were combined and concentrated to afford a crude product. Afterwards, the crude product was purified by normal phase column chromatography (ethyl acetate: petroleum ether = 0% to 50%) to afford a yellow oily product **39-5** (200 mg, yield: 28.9%), MS(ESI, m/z): 273.8[M+H]⁺.

Compound **39-5** (200 mg, 0.73 mmol) was added to dichloromethane (10 mL). Under the ice-bath condition, boron tribromide (1 mL) was slowly added to the reaction solution and stirred at 0°C for 30 min. Upon completion of the reaction, methanol (1 mL) was added to quench the reaction. The reaction solution was concentrated to afford a crude product. Afterwards, the crude product was purified by normal phase column chromatography (ethyl acetate: petroleum ether = 0% to 50%) to afford compound **39-6** (76 mg, yield: 67.9%) as a yellow solid, MS(ESI, m/z): 153.8[M+H]⁺.

Compound **39-6** (76 mg, 0.50 mmol), compound **1-2** (190 mg, 0.50 mmol), cuprous iodide (94 mg, 0.50 mmol), (1R,2R)-(-)-N,N'-dimethyl-1,2-cyclohexanediamine (71 mg, 0.50 mmol), and potassium phosphate (210 mg, 0.99 mmol) were added to N,N-dimethylformamide (5 mL) and heated to 120°C and stirred for 3 hours. Upon completion of the reaction, the reaction solution was directly concentrated, and then purified by reverse-phase column chromatography [acetonitrile/water (containing 0.05% aqueous ammonia) = 0% to 100%] to afford compound **39** (32.1 mg, yield: 14.3%) as a yellow solid, MS(ESI, m/z): 454.8[M+H]⁺.

¹H NMR (400 MHz, DMSO-*d*6) δ 12.43 (s, 1H), 8.22 (d, *J* = 8.0 Hz, 1H), 7.85 - 7.75 (m, 4H), 7.55 (d, *J* = 8.4 Hz, 1H), 7.43 (t, *J* = 7.6 Hz, 1H), 6.07 (d, *J* = 7.2 Hz, 1H), 5.64 (s, 2H), 2.26 - 2.15 (m, 1H), 1.03 - 0.89 (m, 4H).

### Example 38: Synthesis of Compound 40

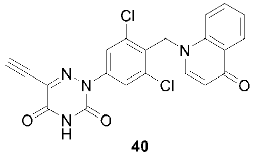

### Synthetic route:

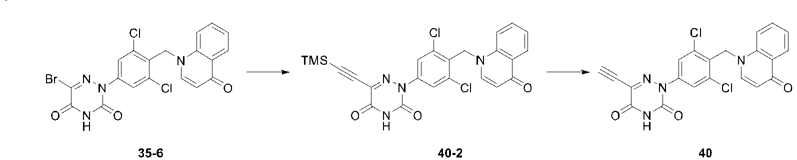

Compound **35-6** (27 mg, 0.055 mmol), trimethylethynylsilane (43 mg, 0.44 mmol), cuprous iodide (5 mg, 0.027 mmol), and [1,1'-bis(diphenylphosphino)ferrocene]dichloropalladium (8 mg, 0.01 mmol) were added to triethylamine (3 mL) and N,N-dimethylformamide (0.5 mL), and heated to 80°C and stirred for 1 hour under nitrogen atmosphere. Upon completion of the reaction, the reaction solution was directly concentrated, and then purified by normal phase column chromatography (ethyl acetate: petroleum ether = 0% to 50%) to afford compound **40-2** (10 mg, yield: 35.8%) as a yellow solid, MS(ESI, m/z): 510.8[M+H]⁺.

Compound **40-2** (10 mg, 0.02 mmol) and potassium carbonate (11 mg, 0.078 mmol) were added to methanol (4 mL) and N,N-dimethylformamide (1 mL), heated to 50°C, and stirred for 1 hour. Upon completion of the reaction, the reaction solution was directly concentrated, and then purified by reverse-phase column chromatography [acetonitrile/water (containing 0.05% aqueous ammonia) = 0% to 100%] to afford compound **40** (1.7 mg, yield: 19.9%) as a white solid, MS(ESI, m/z): 438.8[M+H]⁺.

¹H NMR (400 MHz, DMSO-*d*6) δ 12.74 (s, 1H), 8.21 (d, *J* = 8.0 Hz, 1H), 7.83 - 7.76 (m, 4H), 7.55 (d, *J =* 8.4 Hz, 1H), 7.42 (t, *J =* 7.6 Hz, 1H), 6.06 (d, *J =* 7.6 Hz, 1H), 5.65 (s, 2H), 4.72 (s, 1H).

### Example 39: Synthesis of Compound 41

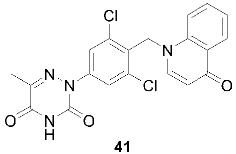

### Synthetic route:

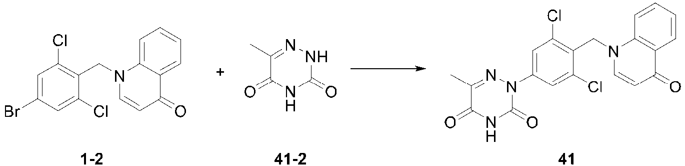

With the exception of replacing compound **1-3** with compound **41-2,** the synthetic route of compound 1 was followed to synthesize compound **41,** which was then purified by reverse-phase column chromatography [acetonitrile/water (containing 0.05% aqueous ammonia) = 0% to 100%] to afford a yellow solid (16.3 mg, yield: 13.3%), MS(ESI, m/z): 428.8[M+H]⁺.

¹H NMR (400 MHz, DMSO-d6) δ 12.45 (s, 1H), 8.23 (d, *J* = 8.0 Hz, 1H), 7.89 - 7.79 (m, 4H), 7.62 (d, *J =* 8.0 Hz, 1H), 7.46 (t, *J* = 7.2 Hz, 1H), 6.14 (d, *J =* 8.0 Hz, 1H), 5.68 (s, 2H), 2.17 (s, 3H).

### Example 40: Synthesis of Compound 43

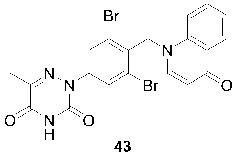

### Synthetic route:

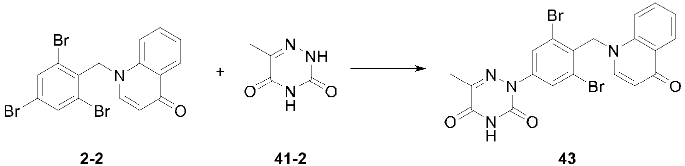

With the exception of replacing compound **1-2** with compound **2-2,** the synthetic route of compound **41** was followed to synthesize compound **43,** which was then purified by reverse-phase column chromatography [acetonitrile/water (containing 0.05% aqueous ammonia) = 0% to 100%] to afford a white solid (8.2 mg, yield: 7.1%), MS(ESI, m/z): 518.8[M+H]⁺.

¹H NMR (400 MHz, DMSO-d6) δ 12.47 (s, 1H), 8.23 (d, *J* = 8.0 Hz, 1H), 8.05 (s, 2H), 7.97 (d, *J* = 8.8 Hz, 1H), 7.82 (t, *J =* 8.0 Hz, 1H), 7.46 (t, *J =* 7.6 Hz, 1H), 7.32 (d, *J =* 8.0 Hz, 1H), 6.06 (d, *J* = 8.0 Hz, 1H), 5.61 (s, 2H), 2.18 (s, 3H).

### Example 41: Synthesis of Compound 44

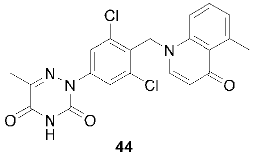

### Synthetic route:

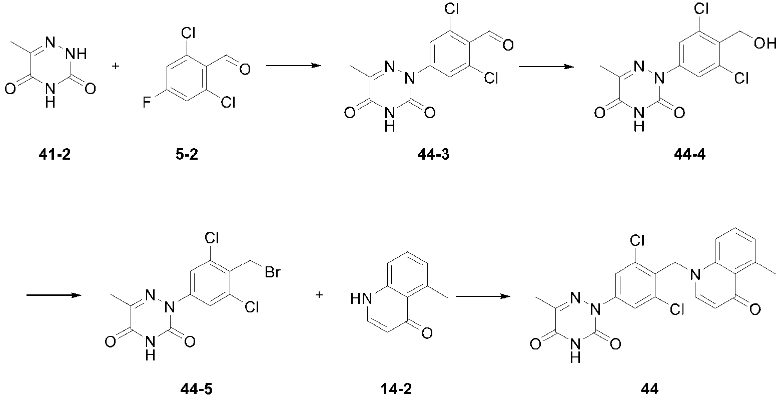

With the exception of replacing compound **28-1** with compound **41-2** and replacing compound **1-1** with compound **14-2,** the synthetic route of compound **28** was followed to synthesize compound **44,** which was then purified by reverse-phase column chromatography [acetonitrile/water (containing 0.05% aqueous ammonia) = 0% to 100%] to afford a white solid (18.4 mg, yield: 19.7%), MS(ESI, m/z): 442.8[M+H]⁺.

¹H NMR (400 MHz, DMSO-*d*6) δ 12.44 (s, 1H), 7.83 (s, 2H), 7.62 - 7.53 (m, 2H), 7.43 (d, *J= 8.0* Hz, 1H), 7.12 (d, *J* = 5.6 Hz, 1H), 5.98 (d, *J=* 8.0 Hz, 1H), 5.56 (s, 2H), 2.82 (s, 3H), 2.17 (s, 3H).

### Example 42: Synthesis of Compound 45

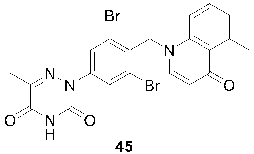

### Synthetic route:

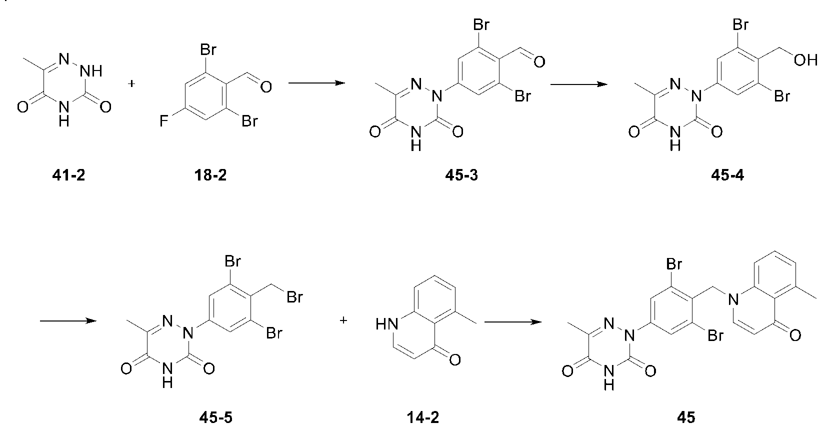

With the exception of replacing compound **1-3** with compound **41-2,** the synthetic route of compound **18** was followed to synthesize compound **45,** which was then purified by reverse-phase column chromatography [acetonitrile/water (containing 0.05% aqueous ammonia) = 0% to 100%] to afford a white solid (15.1 mg, yield: 12.8%), MS(ESI, m/z): 532.8[M+H]⁺.

¹H NMR (400 MHz, DMSO-*d*6) δ 12.46 (s, 1H), 8.04 (s, 2H), 7.74 (d, *J* = 8.8 Hz, 1H), 7.62 (t, *J= 8.0* Hz, 1H), 7.17 (t, *J=* 7.6 Hz, 2H), 5.96 (d, *J* = 8.0 Hz, 1H), 5.51 (s, 2H), 2.83 (s, 3H), 2.18 (s, 3H).

### Example 43: Synthesis of Compound 46

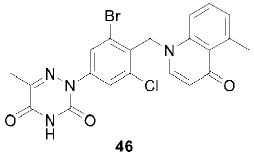

### Synthetic route:

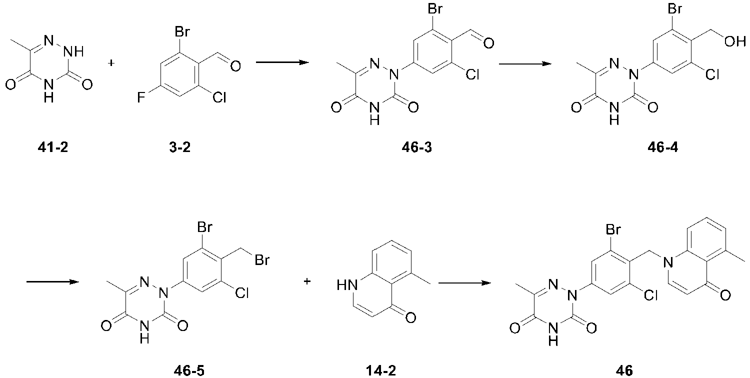

With the exception of replacing compound **5-2** with compound **3-2,** the synthetic route of compound **44** was followed to synthesize compound **46,** which was then purified by reverse-phase column chromatography [acetonitrile/water (containing 0.05% aqueous ammonia) = 0% to 100%] to afford a white solid(20.7 mg, yield: 20.3%), MS(ESI, m/z): 486.8[M+H]⁺.

¹H NMR (400 MHz, DMSO-*d*6) δ 12.43 (s, 1H), 8.01 (s, 1H), 7.88 (s, 1H), 7.68 - 7.57 (m, 2H), 7.30 (d, *J* = 8.0 Hz, 1H), 7.15 (d, *J* = 7.2 Hz, 1H), 5.97 (d, *J* = 8.0 Hz, 1H), 5.53 (s, 2H), 2.83 (s, 3H), 2.18 (s, 3H).

### Example 44: Synthesis of Compound 47

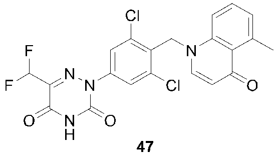

### Synthetic route:

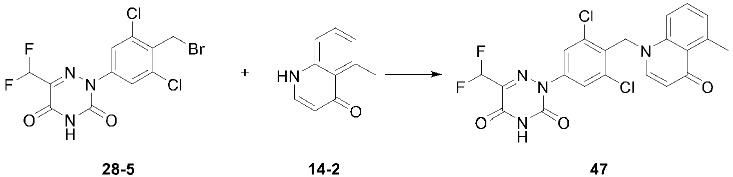

With the exception of replacing compound **1-1** with compound **14-2,** the synthetic route of compound **28** was followed to synthesize compound **47,** which was then purified by reverse-phase column chromatography [acetonitrile/water (containing 0.05% aqueous ammonia) = 0% to 100%] to afford a white solid (100.2 mg, yield: 29.2%), MS(ESI, m/z): 478.8[M+H]⁺.

¹H NMR (400 MHz, DMSO-*d*6) δ 12.86 (s, 1H), 7.85 - 7.73 (m, 2H), 7.58 - 7.54 (m, 2H), 7.47 - 7.42 (m, 1H), 7.17 - 7.07 (m, 1H), 6.91 (t, *J* = 52.4 Hz, 1H), 6.01 - 5.90 (m, 1H), 5.57 (s, 2H), 2.81 (s, 3H).

### Example 45: Synthesis of Compound 48

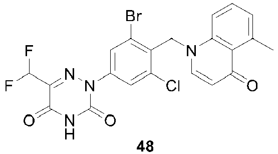

### Synthetic route:

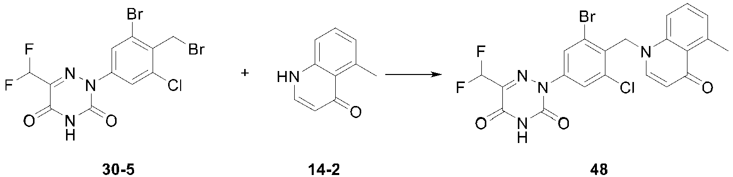

With the exception of replacing compound **1-1** with compound **14-2,** the synthetic route of compound **30** was followed to synthesize compound **48,** which was then purified by reverse-phase column chromatography [acetonitrile/water (containing 0.05% aqueous ammonia) = 0% to 100%] to afford a white solid (20.1 mg, yield: 11.4%), MS(ESI, m/z): 522.8[M+H]⁺.

¹H NMR (400 MHz, DMSO-d6) δ 7.98 (s, 1H), 7.86 (s, 1H), 7.69 - 7.63 (m, 1H), 7.63 - 7.55 (m, 1H), 7.32 (d, *J =* 8.0 Hz, 1H), 7.15 (d, *J =* 7.2 Hz, 1H), 7.09 - 6.74 (m, 1H), 5.97 (d, *J* = 8.0 Hz, 1H), 5.55 (s, 2H), 2.83 (s, 3H).

### Example 46: Synthesis of Compound 49

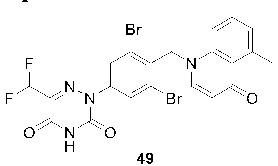

### Synthetic route:

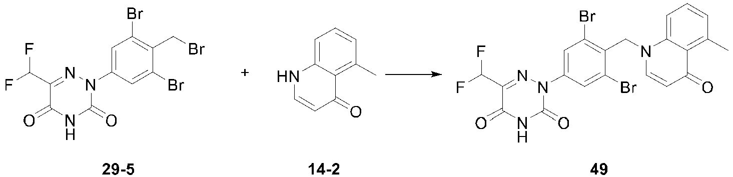

With the exception of replacing compound **1-1** with compound **14-2,** the synthetic route of compound **29** was followed to synthesize compound **49,** which was then purified by reverse-phase column chromatography [acetonitrile/water (containing 0.05% aqueous ammonia) = 0% to 100%] to afford a white solid (25.1 mg, yield: 27.5%), MS(ESI, m/z): 568.8[M+H]⁺.

¹H NMR (400 MHz, DMSO-d6) δ 8.02 (s, 2H), 7.78 - 7.68 (m, 1H), 7.62 (t, *J =* 8.0 Hz, 1H), 7.20 (d, *J* = 8.0 Hz, 1H), 7.16 (d, *J* = 7.6 Hz, 1H), 6.93 (t, *J* = 52.4 Hz, 1H), 5.97 (d, *J* = 8.0 Hz, 1H), 5.52 (s, 2H), 2.83 (s, 3H).

### Example 47: Synthesis of Compound 50

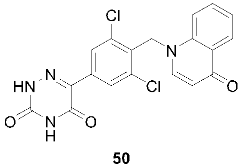

### Synthetic route:

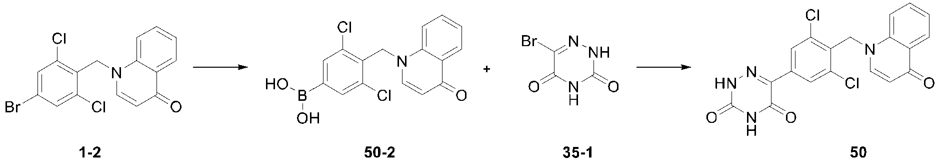

Compound **1-2** (1.6 g, 4.18 mmol), bis(pinacolato)diboron (1 g, 5.01 mmol), [1,1-bis(diphenylphosphino)ferrocene]dichloropalladium (305 mg, 0.42 mmol), and potassium acetate (819 mg, 8.36 mmol) were dissolved into 1,4-dioxane (25 mL) solution, and heated to 100°C and reacted overnight under nitrogen protection. Upon completion of the reaction, the reaction solution was directly concentrated, and then purified by normal phase column chromatography (ethyl acetate: petroleum ether = 0% to 50%) to afford a yellow oily product **50-2** (800.4 mg, yield: 55.2%). MS(ESI, m/z): 347.8[M+H]⁺.

Compound **50-2** (122 mg, 0.35 mmol), compound **35-1** (80 mg, 0.42 mmol), tetrakis(triphenylphosphine)palladium (41 mg, 0.035 mmol), and sodium carbonate (111 mg, 1.05 mmol) were dissolved into a mixed solution of 1,4-dioxane (4 mL) and water (0.5 mL), and heated to 100°C and reacted overnight under nitrogen protection. Upon completion of the reaction, the reaction solution was directly concentrated, and then purified by reverse-phase column chromatography [acetonitrile/water (containing 0.05% aqueous ammonia) = 0% to 100%] to afford compound **50** (2.8 mg, yield: 1.9%) as a yellow solid. MS(ESI, m/z): 414.8[M+H]⁺.

¹H NMR (400 MHz, DMSO-d6) δ 8.21 (d, *J =* 8.0 Hz, 1H), 8.07 (s, 2H), 7.83 - 7.75 (m, 2H), 7.57 (d, *J =* 8.0 Hz, 1H), 7.43 (t, *J* = 7.2 Hz, 1H), 6.07 (d, *J* = 8.0 Hz, 1H), 5.66 (s, 2H).

### Example 48: Synthesis of Compound 51

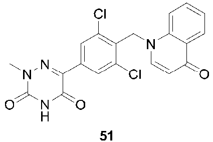

### Synthetic route:

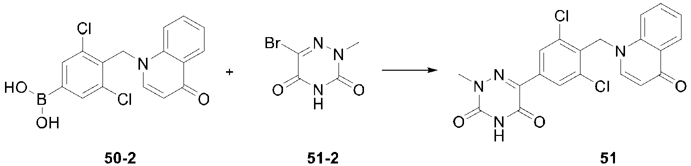

With the exception of replacing compound **35-1** with compound **51-2**, the synthetic route of compound **50** was followed to synthesize compound **51**, which was then purified by reverse-phase column chromatography [acetonitrile/water (containing 0.05% aqueous ammonia) = 0% to 100%] to afford a white solid(9.6 mg, yield: 7.8%), MS(ESI, m/z): 428.8[M+H]⁺.

¹H NMR (400 MHz, DMSO-d6) δ 12.47 (s, 1H), 8.21 (d, *J =* 8.0 Hz, 1H), 8.13 (s, 2H), 7.85 - 7.71 (m, 2H), 7.57 (d, *J =* 7.6 Hz, 1H), 7.46 - 7.38 (m, 1H), 6.06 (d, *J =* 8.0 Hz, 1H), 5.67 (s, 2H), 3.58 (s, 3H).

### Example 49: Synthesis of Compound 52

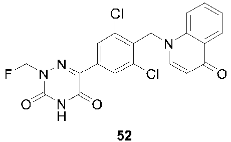

### Synthetic route:

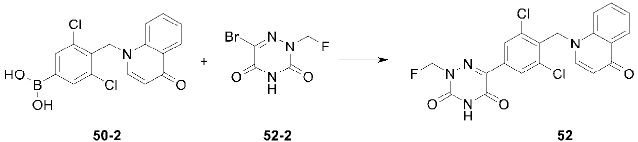

With the exception of replacing compound **35-1** with compound **52-2**, the synthetic route of compound **50** was followed to synthesize compound **52**, which was then purified by reverse-phase column chromatography [acetonitrile/water (containing 0.05% aqueous ammonia) = 0% to 100%] to afford a white solid (9.2 mg, yield: 12.9%), MS(ESI, m/z): 446.8[M+H]⁺.

¹H NMR (400 MHz, DMSO-d6) δ 8.21 (d, *J =* 8.0 Hz, 1H), 8.11 (s, 2H), 7.84 - 7.73 (m, 2H), 7.58 (d, *J* = 8.0 Hz, 1H), 7.42 (t, *J =* 7.6 Hz, 1H), 6.06 (d, *J* = 9.2 Hz, 2H), 5.92 (s, 1H), 5.68 (s, 2H).

### Example 50: Synthesis of Compound 54

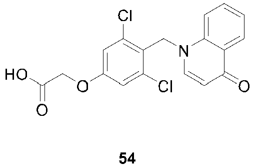

### Synthetic route:

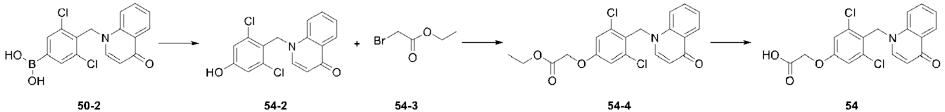

Compound **50-2** (349 mg, 1 mmol) was dissolved into a mixed solution of tetrahydrofuran (3 mL) and methanol (3 mL). Thereafter, sodium hydroxide (100 mg, 2.5 mmol) and hydrogen peroxide (2.5 mL) were slowly added to the reaction solution and stirred at room temperature for one hour. Upon completion of the reaction, a saturated sodium bisulfite solution was added, and the organic phases were concentrated and purified by normal phase column chromatography (ethyl acetate: petroleum ether = 0% to 50%) to afford compound **54-2** (250.3 mg, yield: 77.9%) as a white solid. MS(ESI, m/z): 319.8[M+H]⁺.

Compound **54-2** (90 mg, 0.28 mmol), compound **54-3** (70 mg, 0.42 mmol), potassium carbonate (78 mg, 0.56 mmol), and sodium iodide (42 mg, 0.28 mmol) were added to a mixed solution of N,N-dimethylformamide (1 mL) and acetonitrile (2 mL), heated to 80°C, and reacted overnight. Upon completion of the reaction, the reaction solution was directly concentrated, and then purified by normal phase column chromatography (ethyl acetate: petroleum ether = 0% to 50%) to afford a yellow oily product **54-4** (80.2 mg, yield: 35.7%). MS(ESI, m/z): 406.1[M+H]⁺.

Compound **54-4** (80 mg, 0.2 mmol) was added to a mixed solution of tetrahydrofuran (2 mL) and methanol (2 mL). Thereafter, a solution of lithium hydroxide (14 mg, 0.6 mmol) in water (0.5 mL) was added to the reaction solution and reacted at room temperature for 2 hours. Upon completion of the reaction, the reaction solution was directly concentrated, and then purified by reverse-phase column chromatography [acetonitrile/water = 50% to 100%] to afford compound **54** (20.1 mg, yield: 27.0%) as a white solid. MS(ESI, m/z): 377.8[M+H]⁺.

¹H NMR (400 MHz, DMSO-d6) δ 8.21 (d, *J* = 8.0 Hz, 1H), 7.90 (d, *J* = 8.8 Hz, 1H), 7.77 (t, *J = 7.6* Hz, 1H), 7.45 - 7.41 (m, 2H), 7.26 (s, 2H), 6.05 (d, *J* = 8.0 Hz, 1H), 5.51 (s, 2H), 4.82 (s, 2H).

### Example 51: Synthesis of Compound 55

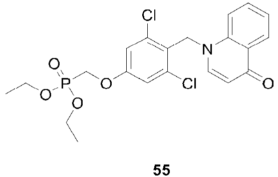

### Synthetic route:

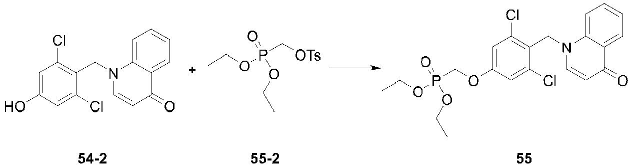

Compound **54-2** (88 mg, 0.28 mmol), compound **55-2** (133 mg, 0.41 mmol), and cesium carbonate (179 mg, 0.55 mmol) were added to a solution of N,N-dimethylformamide (2 mL), heated to 80°C and reacted overnight. Upon completion of the reaction, the reaction solution was directly concentrated, and then purified by reverse-phase column chromatography [acetonitrile/water (containing 0.05% aqueous ammonia) = 0% to 100%] to afford compound **55** (46.1 mg, yield: 35.7%) as a yellow solid. MS(ESI, m/z): 469.8[M+H]⁺.

¹H NMR (400 MHz, DMSO-d6) δ 8.21 (d, *J =* 7.6 Hz, 1H), 7.89 (d, *J* = 8.4 Hz, 1H), 7.77 (t, *J* = 7.2 Hz, 1H), 7.45 - 7.40 (m, 4H), 6.04 (d, *J* = 8.0 Hz, 1H), 5.52 (s, 2H), 4.61 (d, *J* = 10.0 Hz, 2H), 4.16 - 4.09 (m, 4H), 1.25 (t, *J =* 7.2 Hz, 6H).

### Example 52: Synthesis of Compound 56

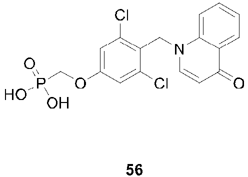

### Synthetic route:

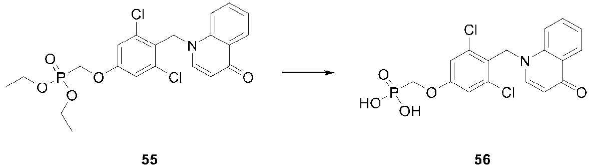

Compound **55** (30 mg, 0.064 mmol) was added to a solution of dichloromethane (5 mL). Thereafter, bromotrimethylsilane (1 mL) was added to the reaction solution and reacted at room temperature for 72 hours. Upon completion of the reaction, the reaction solution was directly concentrated, and then purified by reverse-phase column chromatography [acetonitrile/water = 50% to 100%] to afford compound **56** (12.4 mg, yield: 47.0%) as a white solid. MS(ESI, m/z): 413.8[M+H]⁺.

¹H NMR (400 MHz, DMSO-d6) δ 8.21 (d, *J* = 7.6 Hz, 1H), 7.91 (d, *J* = 8.8 Hz, 1H), 7.79 (t, *J* = 7.6 Hz, 1H), 7.46 - 7.40 (m, 2H), 7.34 (s, 2H), 6.05 (d, *J =* 8.0 Hz, 1H), 5.52 (s, 2H), 4.27 (d, *J* = 10.0 Hz, 2H).

### Example 53: Synthesis of Compound 57

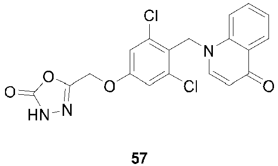

### Synthetic route:

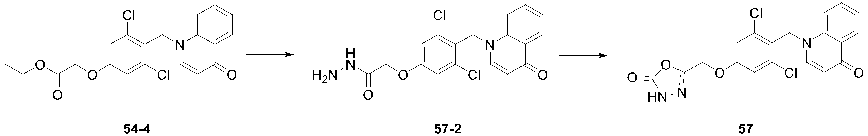

Compound **54-4** (133 mg, 0.33 mmol) and hydrazine hydrate (2 mL) were added to an ethanol (10 mL) solution, heated to 85°C, and reacted for two hours. Upon completion of the reaction, the reaction solution was directly concentrated, and then purified by normal phase column chromatography (ethyl acetate: petroleum ether = 0% to 50%) to afford a yellow oily product **57-2** (100.2 mg, yield: 78.1%). MS(ESI, m/z): 391.9[M+H]⁺.

Compound **57-2** (100 mg, 0.26 mmol), N,N'-disuccinimidyl carbonate (196 mg, 0.77 mmol), and triethylamine (77 mg, 0.77 mmol) were added to a tetrahydrofuran (10 mL) solution, heated to 85°C, and reacted for three hours. After the reaction solution was cooled to room temperature and directly filtered, the filter cake was dried, and then purified by reverse-phase column chromatography [acetonitrile/water = 0% to 100%] to afford compound 57 (52.1 mg, yield: 48.9%) as a gray solid. MS(ESI, m/z): 417.8[M+H]⁺.

¹H NMR (400 MHz, DMSO-d6) δ 8.21 (d, *J* = 8.0 Hz, 1H), 7.89 (d, *J* = 8.4 Hz, 1H), 7.77 (t, *J =* 7.6 Hz, 1H), 7.46 - 7.38 (m, 4H), 6.04 (d, *J* = 7.6 Hz, 1H), 5.52 (s, 2H), 5.22 (s, 2H).

### Example 54: Synthesis of Compound 58

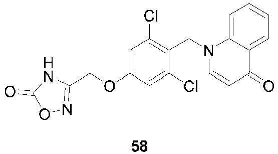

### Synthetic route:

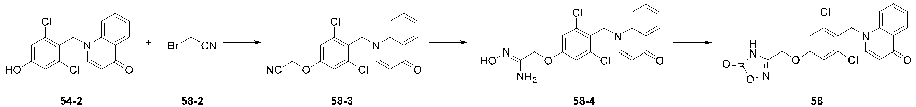

Compound **54-2** (490 mg, 1.53 mmol), compound **58-2** (276 mg, 2.3 mmol), potassium carbonate (423 mg, 3.06 mmol), and sodium iodide (230 mg, 1.53 mmol) were added to a mixed solution of N,N-dimethylformamide (3 mL) and acetonitrile (3 mL), heated to 80°C, and reacted for six hours. The reaction solution was cooled to room temperature. The reaction solution was directly concentrated, and then purified by normal phase column chromatography (ethyl acetate: petroleum ether = 0% to 50%) to afford compound **58-3** (545.2 mg, yield: 99.5%) as a yellow solid. MS(ESI, m/z): 358.8[M+H]⁺.

Compound **58-3** (480 mg, 1.343 mmol), hydroxylamine hydrochloride (232 mg, 3.34 mmol), and triethylamine (338 mg, 3.34 mmol) were added to ethanol (20 mL), heated to 90°C, and reacted for three hours. The reaction solution was cooled to room temperature. The reaction solution was directly concentrated, and then purified by normal phase column chromatography (ethyl acetate: petroleum ether = 0% to 50%) to afford a yellow oily product **58-4** (179.1 mg, yield: 34.2%). MS(ESI, m/z): 391.9[M+H]⁺.

Compound **58-4** (179 mg, 0.46 mmol), N,N'-disuccinimidyl carbonate (351 mg, 1.37 mmol), and triethylamine (231 mg, 2.28 mmol) were added to tetrahydrofuran (10 mL), heated to 80°C, and reacted for five hours. Upon completion of the reaction, the reaction solution was directly concentrated, and then purified by reverse-phase column chromatography [acetonitrile/water = 0% to 100%] to afford compound 58 (40.4 mg, yield: 21.2%) as a gray solid. MS(ESI, m/z): 417.8[M+H]⁺.

¹H NMR (400 MHz, DMSO-d6) δ 8.21 (d, *J* = 8.0 Hz, 1H), 7.89 (d, *J* = 8.8 Hz, 1H), 7.77 (t, *J =* 7.6 Hz, 1H), 7.45 - 7.41 (m, 4H), 6.05 (d, *J* = 8.0 Hz, 1H), 5.53 (s, 2H), 5.22 (s, 2H).

### Example 55: Synthesis of Compound 59

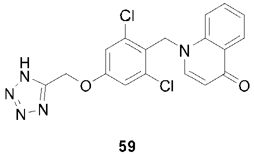

### Synthetic route:

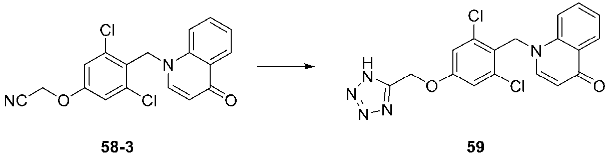

Compound **58-3** (150 mg, 0.42 mmol), cuprous oxide (1.5 mg, 0.01 mmol), and azidotrimethylsilane (72 mg, 0.63 mmol) were added to a mixed solution of N,N-dimethylformamide (0.9 mL) and methanol (0.1 mL), stirred at room temperature for 15 min, and then heated to 80°C and reacted for five hours. Upon completion of the reaction, the reaction solution was directly concentrated, and then purified by reverse-phase column chromatography [acetonitrile/water (containing 0.05% aqueous ammonia) = 0% to 100%] to afford compound **59** (35.6 mg, yield: 21.2%) as a gray solid. MS(ESI, m/z): 401.8[M+H]⁺.

¹H NMR (400 MHz, DMSO-d6) δ 8.21 (d, *J* = 8.0 Hz, 1H), 7.90 (d, *J* = 8.8 Hz, 1H), 7.78 (t, *J =* 7.6 Hz, 1H), 7.54 - 7.35 (m, 4H), 6.05 (d, *J* = 7.6 Hz, 1H), 5.73 - 5.46 (s, 4H).

### Example 56: Synthesis of Compound 60

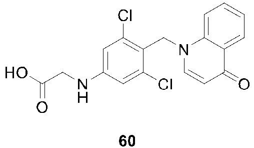

### Synthetic route:

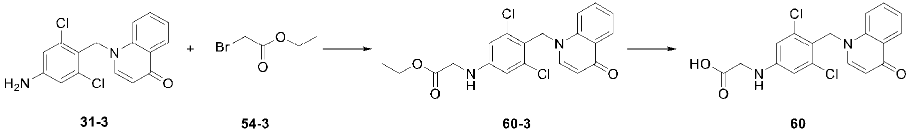

With the exception of replacing compound **54-2** with compound **31-3**, the synthetic route of compound **54** was followed to synthesize compound **60**, which was then purified by reverse-phase column chromatography [acetonitrile/water = 50% to 100%] to afford a white solid (5.7 mg, yield: 17.5%), MS(ESI, m/z): 376.8[M+H]⁺.

¹H NMR (400 MHz, DMSO-d6) δ 8.21 (d, *J =* 7.6 Hz, 1H), 7.96 (d, *J* = 8.8 Hz, 1H), 7.78 (t, *J =* 7.6 Hz, 1H), 7.43 (t, *J* = 7.6 Hz, 1H), 7.36 (d, *J* = 7.6 Hz, 1H), 6.83 - 6.78 (m, 3H), 6.05 (d, *J =* 8.0 Hz, 1H), 5.39 (s, 2H), 3.92 (d, *J* = 5.2 Hz, 2H).

### Example 57: Synthesis of Compound 61

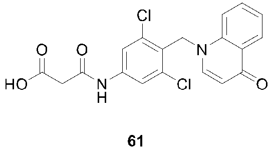

### Synthetic route:

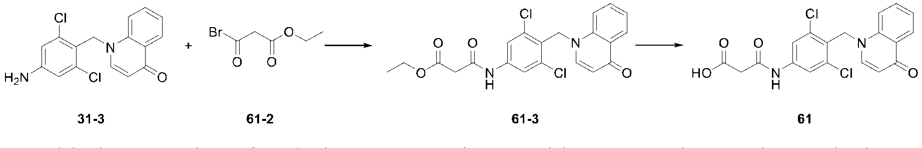

With the exception of replacing compound **54-3** with compound 61-2, the synthetic route of compound **60** was followed to synthesize compound **61**, which was then purified by reverse-phase column chromatography [acetonitrile/water = 50% to 100%] to afford a white solid (39.8 mg, yield: 53.2%), MS(ESI, m/z): 404.8[M+H]⁺.

¹H NMR (400 MHz, DMSO-d6) δ 10.64 (s, 1H), 8.21 (d, *J =* 7.6 Hz, 1H), 7.88 - 7.75 (m, 4H), 7.48 - 7.41 (m, 2H), 6.03 (d, *J* = 7.6 Hz, 1H), 5.54 (s, 2H), 3.40 (s, 2H).

### Example 58: Synthesis of Compound 62

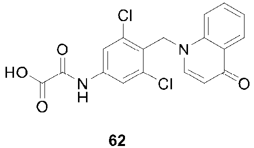

### Synthetic route:

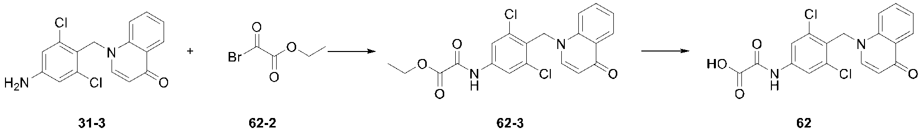

With the exception of replacing compound **54-3** with compound **62-2**, the synthetic route of compound **60** was followed to synthesize compound **62**, which was then purified by reverse-phase column chromatography [acetonitrile/water = 50% to 100%] to afford a white solid (8.5 mg, yield: 15.2%). MS(ESI, m/z): 390.8[M+H]⁺.

¹H NMR (400 MHz, DMSO-d6) δ 11.17 (s, 1H), 8.21 (d, *J* = 8.0 Hz, 1H), 8.08 (s, 2H), 7.86 (d, *J =* 8.4 Hz, 1H), 7.77 (t, *J* = 7.6 Hz, 1H), 7.49 (d, *J* = 8.0 Hz, 1H), 7.43 (t, *J* = 7.2 Hz, 1H), 6.04 (d, *J* = 8.0 Hz, 1H), 5.56 (s, 2H).

### Example 59: Synthesis of Compound 63

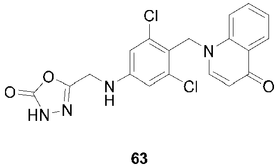

### Synthetic route:

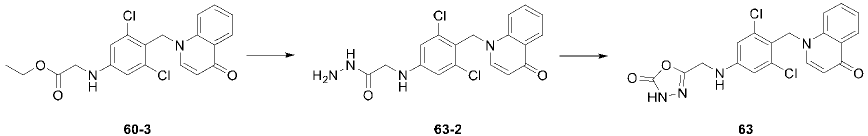

With the exception of replacing compound **54-4** with compound **60-3**, the synthetic route of compound **57** was followed to synthesize compound **63**, which was then purified by reverse-phase column chromatography [acetonitrile/water = 0% to 100%] to afford a white solid (10.1 mg, yield: 18.8%). MS(ESI, m/z): 416.8[M+H]⁺.

¹H NMR (400 MHz, DMSO-d6) δ 12.28 (s, 1H), 8.21 (d, *J =* 8.0 Hz, 1H), 7.94 (d, *J* = 8.8 Hz, 1H), 7.83 - 7.71 (m, 1H), 7.43 (t, *J* = 7.6 Hz, 1H), 7.38 (d, *J* = 8.0 Hz, 1H), 7.16 - 7.07 (m, 1H), 6.90 (s, 2H), 6.09 - 6.00 (m, 1H), 5.40 (s, 2H), 4.35 (d, *J* = 6.0 Hz, 2H).

### Example 60: Synthesis of Compound 64

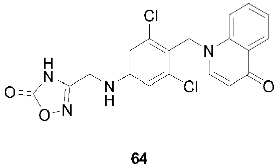

### Synthetic route:

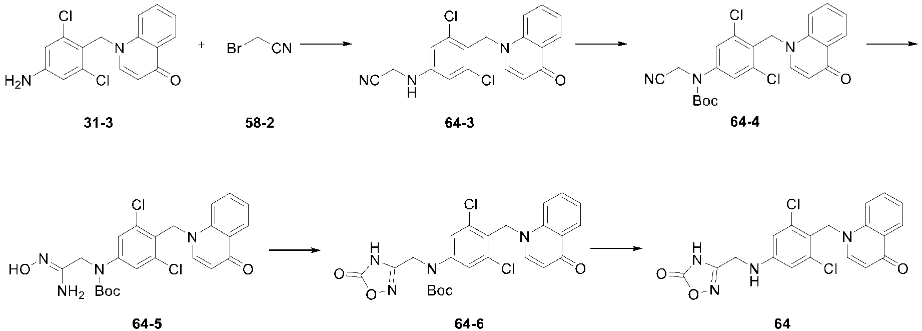

A mixture of compound **31-3** (100 mg, 0.314 mmol), bromoacetonitrile **58-2** (377 mg, 3.14 mmol), sodium iodide (141 mg, 0.942 mmol), potassium carbonate (87 mg, 0.628 mmol), and DMSO (2 mL) was stirred at 70°C for 16 hours. The reaction solution was diluted with water (30 mL), and extracted with ethyl acetate (30 mL×2). The organic phases were directly concentrated, and then purified by normal phase column chromatography (ethyl acetate: petroleum ether = 0% to 50%) to afford compound **64-3** (111.5 mg, yield: 99.5%) as a black solid. MS(ESI, m/z): 357.8[M+H]⁺.

A mixture of compound **64-3** (110 mg, 0.31 mmol), di-tert-butyl dicarbonate (119 mg, 0.55 mmol), 4-dimethylaminopyridine (5 mg, 0.042 mmol), triethylamine (125 mg, 1.24 mmol), and tetrahydrofuran (3 mL) was stirred at room temperature for 16 hours. The reaction solution was directly concentrated, and then purified by normal phase column chromatography (ethyl acetate: petroleum ether = 0% to 50%) to afford compound **64-4** (82.4 mg, yield: 34.8%) as a yellow solid. MS(ESI, m/z): 457.8[M+H]⁺.

A mixture of compound **64-4** (82 mg, 0.18 mmol), an aqueous hydroxylamine solution (1 mL), and ethanol (2 mL) was stirred at 95°C for 2 hours. The reaction solution was directly concentrated to afford compound **64-5** (80 mg, crude product) as a yellow oily product. MS(ESI, m/z): 490.8[M+H]⁺.

A mixture of compound **64-5** (80 mg, 0.163 mmol), N,N'-disuccinimidyl carbonate (125 mg, 0.489 mmol), triethylamine (49 mg, 0.489 mmol), and tetrahydrofuran (2 mL) was stirred at 95°C for 1 hour. The reaction solution was directly concentrated, and then purified by reverse-phase column chromatography [acetonitrile/water (containing 0.05% aqueous ammonia) = 0% to 100%] to afford compound **64-6** (20.1 mg, yield: 23.3%) as a white solid. MS(ESI, m/z): 516.8[M+H]⁺.

To a solution of compound **64-6** (15 mg, 0.029 mmol) in dichloromethane (2 mL), a solution (4M, 2 mL) of hydrochloric acid in dioxane was added dropwise. The reaction solution was stirred at room temperature for 2 hours. The reaction solution was directly concentrated, and then purified by reverse-phase column chromatography [acetonitrile/water = 0% to 100%] to afford compound **64** (2.3 mg, yield: 19.1%) as a white solid. MS(ESI, m/z): 416.8[M+H]⁺.

¹H NMR (400 MHz, DMSO-d6) δ 8.25 - 8.16 (m, 1H), 7.95 (d, *J* = 8.4 Hz, 1H), 7.84 - 7.69 (m, 1H), 7.47 - 7.40 (m, 1H), 7.37 (d, J = 8.0 Hz, 1H), 7.11 - 6.97 (m, 1H), 6.88 (s, 2H), 6.05 (d, *J =* 8.0 Hz, 1H), 5.41 (s, 2H), 4.35 (d, J = 6.0 Hz, 2H).

### Example 61: Synthesis of Compound 65

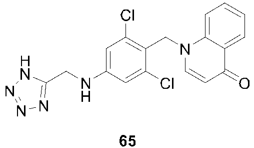

### Synthetic route:

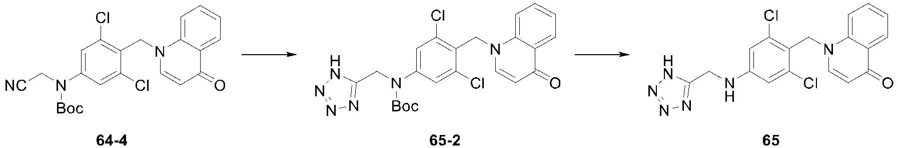

A mixture of compound **64-4** (124 mg, 0.271 mmol), cuprous oxide (1 mg, 0.007 mmol), N,N-dimethylformamide (0.9 mL), methanol (0.1 mL), and azidotrimethylsilane (62 mg, 0.542 mmol) was stirred at room temperature for 15 min under nitrogen protection, and then stirred at 80°C for 16 hours. The reaction solution was directly concentrated, and then purified by normal phase column chromatography (ethyl acetate: petroleum ether = 0% to 50%) to afford compound **65-2** (20.3 mg, yield: 14.8%) as a white solid. MS(ESI, m/z): 500.8[M+H]⁺.

To a solution of compound **65-2** (20 mg, 0.04 mmol) in dichloromethane (2 mL), a solution (4M, 2 mL) of hydrochloric acid in dioxane was added dropwise. The reaction solution was stirred at room temperature for 2 hours. The reaction solution was concentrated, and then purified by reverse-phase column chromatography [acetonitrile/water (containing 0.05% aqueous ammonia) = 0% to 100%] to afford compound **65** (2.2 mg, yield: 13.6%) as a white solid. MS(ESI, m/z): 400.8[M+H]⁺.

¹H NMR (400 MHz, DMSO-d6) δ 8.21 (d, *J* = 8.0 Hz, 1H), 7.95 (d, *J* = 8.0 Hz, 1H), 7.77 (t, *J =* 8.0 Hz, 1H), 7.43 (t, *J =* 7.6 Hz, 1H), 7.36 (d, *J* = 8.0 Hz, 1H), 7.23 (m, 1H), 6.86 (s, 2H), 6.04 (d, *J* = 8.0 Hz, 1H), 5.40 (s, 2H), 4.70 (s, 2H).

### Example 62: Synthesis of Compound 66

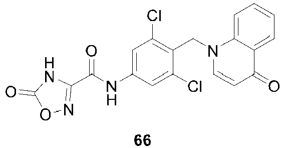

### Synthetic route:

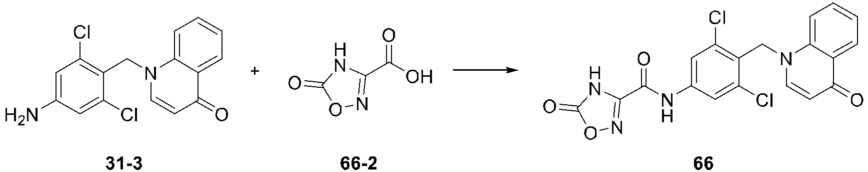

Compound **66-2** (204 mg, 1.57 mmol) was added to tetrahydrofuran (10 mL). Subsequently, under the ice-bath condition, oxalyl chloride (419 mg, 3.3 mmol) and one drop of N,N-dimethylformamide were slowly added to the reaction solution and stirred for one hour. After the reaction solution was concentrated, the resultant was re-dissolved into tetrahydrofuran (5 mL), and then slowly added to a solution of compound **31-3** (175 mg, 0.55 mmol) and triethylamine (166 mg, 1.65 mmol) in N-methylpyrrolidone (10 mL), and heated to 60°C and stirred for two hours. Upon completion of the reaction, the reaction solution was directly concentrated, and then purified by reverse-phase column chromatography [acetonitrile/water (containing 0.05% aqueous ammonia) = 0% to 100%] to afford compound **66** (3.5 mg, yield1.5%) as a white solid. MS(ESI, m/z): 430.8[M+H]⁺.

¹H NMR (400 MHz, DMSO-d6) δ 11.17 (s, 1H), 8.21 (d, *J =* 8.0 Hz, 1H), 8.08 (s, 2H), 7.86 (d, *J =* 8.0 Hz, 1H), 7.77 (t, *J* = 7.6 Hz, 1H), 7.49 (d, *J =* 8.0 Hz, 1H), 7.42 (t, *J* = 6.8 Hz, 1H), 6.04 (d, *J* = 8.0 Hz, 1H), 5.56 (s, 2H).

### Example 63: Synthesis of Compound 70

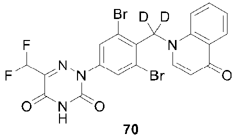

### Synthetic route:

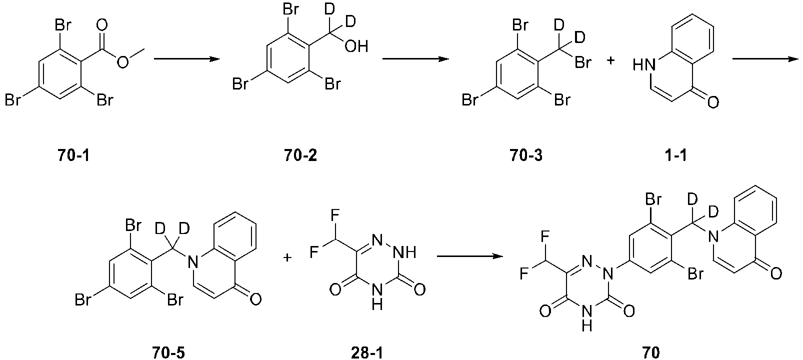

Compound **70-1** (2.8 g, 7.53 mmol) was added to tetrahydrofuran (25 mL). Subsequently, under the ice-bath condition, deuterated lithium aluminum hydride (632 mg, 15.1 mmol) was added to the reaction solution and stirred at room temperature for one hour. Upon completion of the reaction, water (0.6 mL), a 15% sodium hydroxide solution (0.6 mL), and water (1.8 mL) were added successively to quench the system. Afterwards, the system was extracted with ethyl acetate (100 mL). The organic phases were washed with saturated saline, dried over anhydrous sodium sulfate, and then filtered. The organic phases were directly concentrated, and then purified by normal phase column chromatography (ethyl acetate: petroleum ether = 0% to 50%) to afford compound **70-2** (1 g, yield: 34.5%) as a white solid, MS(ESI, m/z): 346.8[M+H]⁺.

Compound **70-2** (253 mg, 0.73 mmol) was added to dichloromethane (15 mL). Subsequently, under the ice-bath condition, phosphorus tribromide (217 mg, 0.80 mmol) was added to the reaction solution and stirred at room temperature for 1 hour. Upon completion of the reaction, the reaction solution was directly concentrated to afford a crude product. Afterwards, the crude product was purified by normal phase column chromatography (ethyl acetate: petroleum ether = 0% to 50%) to afford compound 70-3 (133 mg, yield: 44.3%) as a white solid, MS(ESI, m/z): 410.8[M+H]⁺.

Compound 1-1 (94 mg, 0.65 mmol) and sodium hydride (39 mg, 0.97 mmol) were added to N,N-dimethylformamide (3 mL), and stirred for 30 min under the ice-bath condition. Afterwards, a solution of compound 70-3 (133 mg, 0.32 mmol) in N,N-dimethylformamide (2 mL) was slowly added to the reaction solution and stirred at room temperature for 1 hour. Upon completion of the reaction, the reaction solution was directly concentrated, and then purified by normal phase column chromatography (ethyl acetate: petroleum ether = 0% to 50%) to afford compound **70-5** (70mg, yield: 45.8%) as a white solid, MS(ESI, m/z): 473.8[M+H]⁺.

Compound **70-5** (70 mg, 0.15 mmol), compound **28-1** (29 mg, 0.18 mmol), copper(I) cyanide (13 mg, 0.15 mmol), (1R,2R)-(-)-N,N'-dimethyl-1,2-cyclohexanediamine (21 mg, 0.15 mmol), and potassium carbonate (41 mg, 0.30 mmol) were added to N,N-dimethylformamide (3 mL), and heated to 100°C and stirred overnight. Upon completion of the reaction, the reaction solution was directly concentrated, and then purified by reverse-phase column chromatography [acetonitrile/water (containing 0.05% aqueous ammonia) = 0% to 100%] to afford compound **70** (2.3 mg, yield: 2.8%) as a yellow solid, MS(ESI, m/z): 556.8[M+H]⁺.

¹H NMR (400 MHz, DMSO-d6) δ 12.82 (s, 1H), 8.86 - 8.79 (m, 1H), 8.15 - 7.97 (m, 4H), 7.79 - 7.72 (m, 1H), 7.55 - 7.50 (m, 1H), 7.36 - 7.31 (m, 1H), 6.92 (t, *J =* 52.4 Hz, 1H).

### Example 64: Synthesis of Compound 71

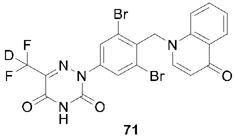

### Synthetic route:

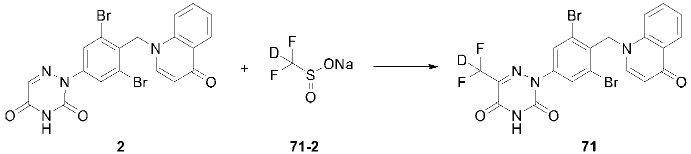

Compound 2 (100 mg, 0.20 mmol), compound 71-2 (55 mg, 0.40 mmol), and Acid Red 94 (4 mg, 0.004 mmol) were added to dimethylsulfoxide (5 mL), and heated to 50°C and stirred for 1 hour under green light. Upon completion of the reaction, the reaction solution was directly concentrated, and then purified by reverse-phase column chromatography [acetonitrile/water (containing 0.05% aqueous ammonia) = 0% to 100%] to afford compound 71 (23.8 mg, yield: 21.6%) as a white solid, MS(ESI, m/z): 555.8[M+H]⁺.

¹H NMR (400 MHz, DMSO-d6) δ 12.87 (s, 1H), 8.23 (dd, *J =* 8.0 Hz, 1.2 Hz, 1H), 8.03 (s, 2H), 7.96 (d, *J* = 8.8 Hz, 1H), 7.86 - 7.77 (m, 1H), 7.46 (t, *J* = 7.6 Hz, 1H), 7.35 (d, *J* = 8.0 Hz, 1H), 6.06 (d, *J* =8.0 Hz, 1H), 5.62 (s, 2H).

### Example 65: Synthesis of Compound 72

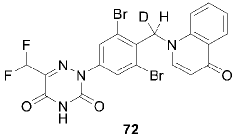

### Synthetic route:

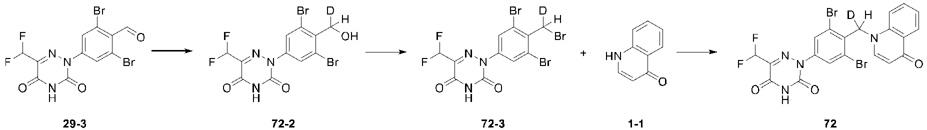

Compound **29-3** (1 g, 2.35 mmol) was added to a mixed solution of tetrahydrofuran (10 mL) and methanol (5 mL). Subsequently, under the ice-bath condition, deuterated sodium borohydride (106 mg, 2.59 mmol) was added to the reaction solution and stirred at room temperature for 15 min. Upon completion of the reaction, a saturated ammonium chloride solution (50 mL) was added to quench the system. Thereafter, the reaction system was extracted with ethyl acetate (100 mL). The organic phases were washed with saturated saline. The organic phases were combined and concentrated to afford a crude product. Afterwards, the crude product was purified by normal phase column chromatography (ethyl acetate: petroleum ether = 0% to 50%) to afford a yellow solid **72-2** (1 g, yield: 99.9%), MS(ESI, m/z): 428.7[M+H]⁺.

Compound **72-2** (1 g, 2.33 mmol) was added to a mixed solution of dichloromethane (15 mL) and tetrahydrofuran (10 mL). Subsequently, under the ice-bath condition, phosphorus tribromide (695 mg, 2.56 mmol) was added to the reaction solution and stirred at room temperature for 1 hour. Upon completion of the reaction, the reaction solution was directly concentrated, and then purified by normal phase column chromatography (ethyl acetate: petroleum ether = 0% to 50%) to afford compound **72-3** (800 mg, yield: 69.6%) as a yellow solid, MS(ESI, m/z): 492.7[M+H]⁺.

Compound **1-1** (100 mg, 0.69 mmol) and sodium hydride (42 mg, 1.04 mmol) were added to N,N-dimethylformamide (3 mL) and tetrahydrofuran(5mL), and stirred for 30 min under the ice-bath condition. Thereafter, a solution of compound 72-3 (170 mg, 0.34 mmol) in N,N-dimethylformamide (2 mL) was slowly added to the reaction solution and stirred at room temperature for 1 hour. Upon completion of the reaction, the reaction solution was directly purified by reverse-phase column chromatography [acetonitrile/water (containing 0.05% aqueous ammonia) = 0% to 100%] to afford compound **72** (51.1 mg, yield: 26.6%) as a white solid, MS(ESI, m/z): 555.8[M+H]⁺.

¹H NMR (400 MHz, DMSO-d6) δ 12.86 (s, 1H), 8.23 (dd, *J =* 8.0 Hz, 1.6 Hz, 1H), 8.03 (s, 2H), 7.96 (d, *J* = 8.8 Hz, 1H), 7.85 - 7.78 (m, 1H), 7.34 (d, *J* = 7.6 Hz, 1H), 6.93 (t, *J* = 52.4 Hz, 1H), 6.06 (d, *J* = 8.0 Hz, 1H), 5.61 (d, *J* = 7.6 Hz, 1H).

### Comparative Example 1: Synthesis of Compound 73

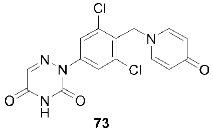

### Synthetic route:

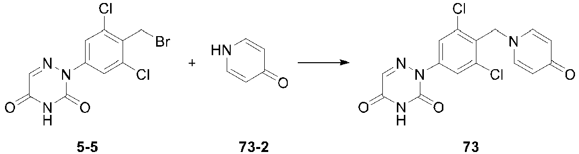

With the exception of replacing compound 5-6 with compound 73-2, the synthetic route of compound 5 was followed to synthesize compound 73, which was then purified by reverse-phase column chromatography [acetonitrile/water (containing 0.05% aqueous ammonia) = 0% to 100%] to afford a white solid (12.5 mg, yield: 16.2%). MS(ESI, m/z): 364.8[M+H]⁺.

¹H NMR (400 MHz, DMSO-d6) δ 12.48 (s, 1H), 7.80 (s, 2H), 7.72 (s, 1H), 7.59 (d, *J* = 6.0 Hz, 2H), 6.09 (d, *J* = 6.0 Hz, 2H), 5.33 (s, 2H).

### Bioactivity Assay

### Experimental Example 1: Detection of Agonist Activities of Compounds Against THRα/β Based on Time-Resolved Fluorescence Resonance Energy Transfer (THR-FRET)

### 1. Construction of THRα/β overexpression vectors

By visiting NCBI, the THRα/β LBD domain sequences were found. The pET21-His-GST-dLBT-THRα LBD and pET21-His-GST-dLBT-THRβ LBD overexpression vectors were constructed by the fusion method, and sequenced to confirm the accuracy of the sequences.

### 2. Prokaryotic expression of recombinant proteins in E. coli

The THRα LBD and THRβ LBD overexpression vectors confirmed by sequencing were transfected into *E. coli* cells BL21 (DE3), and spread on an ampicillin-resistant agar plate. Monoclones were picked out and amplified in an LB medium, and transferred into 1L LB at a ratio of 1:100 for large-scale culture. When the OD value was at 0.8 to 1.2, 0.5 mM isopropyl-beta-D-thiogalactopyranoside (IPTG) was added, and induced overnight at 18°C. The bacteria were harvested, disrupted, and purified via the GST column and the molecular sieve to obtain two proteins His-GST-dLBT-THRα LBD and His-GST-dLBT-THRβ LBD. The protein concentrations were determined to be 24 µM and 23 µM respectively by Bradford Protein Assay Kit of Sangon Biotech.

### 3. Preparation of compounds and formulation of reaction system

The proteins were taken out from a refrigerator at -80°C. The proteins with the GST-tagged THRα/β LBD domain and the Eu-labelled GST antibodies were thawed slowly on ice, and an assay buffer containing a final concentration of 5 mM dithiothreitol (DTT) was formulated.

### 3.1 Preparation of compounds

The starting concentration of the compound was 100 µM (in DMSO). The compound (100 µM in DMSO) was subjected to 3-fold isogradient dilution with DMSO to obtain a total of 11 isogradient concentrations. Subsequently, the above compounds at the isogradient concentration were further subjected to 50-fold dilution with an assay buffer containing 5 mM DTT.

### 3.2 Formulation of THR-FRET reaction system

The final concentrations of all components were calculated based on a system with a final volume of 20 µL per well. To 18 µL of assay buffer containing 5 mM DTT, the GST-tagged THRα/β proteins, the SRC2 (LKEKHKILHRLLQDSSSPV) polypeptide, XL665 (Cisobio, #610SAXLB), and the Eu-labeled GST antibody were added, and the final concentrations were 2 nM, 200 nM, 0.05 nM, and 7.6 nM respectively to formulate 18 µL of reaction mixture of the proteins, polypeptide, and antibody per well.

18 µL of reaction mixture and 2 µL of diluted compound were added into an optiplate-384-well plate, and reacted at room temperature for 24 h.

### 3.3 Plate reading

The plate was read using an MD i3x multi-mode microplate reader with the excitation and emission wavelengths of 340 nm and 665 nm, respectively. The intensity of light at a wavelength of 616 nm generated by europium excited by 340 nm wavelength light from the MD i3x multi-mode microplate reader was used as the background. According to the different activation degrees of different compounds against THRα and THRβ, the intensity of the emission light at a wavelength of 665 nm generated by exciting XL665 with the exciting light at a wavelength of 616 nm was varied. The intensity ratio between these two light wavelengths (665 nm, 616 nm) was used as the activation activity of the compound against THRα or THRβ, and was normalized against the ratio in the vehicle DMSO group. The dose-response curve was fitted with four parameters using the GraphPad Prism 6.0 software, and the EC50 value was calculated.

### 4. Results

Resmetirom (MGL-3196) was an oral liver-targeted and highly selective THR-β agonist, so MGL-3196 was used as a control compound herein for illustrating the bioactivities of the compounds of the present application.

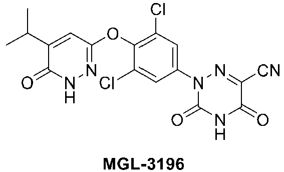

The experimental data suggested that the compounds of the present disclosure exhibited a stronger THRβ agonist activity and certain THRα/β selectivity. The specific data were listed in Table 1.

| **Name of compound** | THRα Agonist Activity EC50 (µM) | THRβ Agonist Activity EC50 (µM) | Selectivity (THRα/THRβ) |
|---|---|---|---|
| **MGL3196** | ** | *** | †† |
| **Compound 1** | ** | *** | †† |
| **Compound 2** | *** | ***** | †† |
| **Compound 3** | *** | *** | †† |
| **Compound 13** | ** | *** | †† |
| **Compound 14** | *** | *** | †† |
| **Compound 17** | *** | ***** | †† |
| **Compound 18** | **** | ***** | †† |
| **Compound 28** | *** | ***** | †† |
| **Compound 29** | **** | ***** | †† |
| **Compound 30** | *** | ***** | †† |
| **Compound 31** | ** | *** | †† |
| **Compound 32** | ** | *** | †† |
| **Compound 35** | ** | *** | †† |
| **Compound 37** | *** | **** | †† |
| **Compound 40** | *** | **** | †† |
| **Compound 41** | ** | *** | †† |
| **Compound 43** | *** | **** | †† |
| **Compound 44** | *** | **** | †† |
| **Compound 45** | *** | ***** | †† |
| **Compound 46** | **** | ***** | †† |
| **Compound 47** | *** | ***** | †† |
| **Compound 48** | ***** | ***** | †† |
| **Compound 49** | **** | **** | †† |
| **Compound 58** | *** | *** | †† |
| **Compound 59** | *** | *** | †† |
| **Compound 60** | *** | *** | †† |
| **Compound 61** | ** | *** | †† |
| **Compound 62** | *** | **** | †† |
| **Compound 63** | ** | *** | †† |
| **Compound 64** | *** | **** | †† |
| **Compound 65** | *** | ***** | ††† |
| **Compound 66** | ** | *** | ††† |
| **Compound 71** | **** | ***** | †† |
| **Compound 72** | **** | ***** | †† |
| **Compound 73** | **NA** | **NA** | **NA** |

| | | | |
|---|---|---|---|
| *: EC50 > 10 µM; **: 10 µM ≥ EC50 > 1 µM; ***: 1 µM ≥ EC50 > 0.1 µM; ****: 0.1 µM ≥ EC50 > 0.05 µM; *****: 0.05 µM ≥ EC50 †: 1 ≥ THRα/β; ††: 10 > THRα/β > 1; †††: THRα/β ≥ 10 NA: No activity | | | |

### Experimental Example 2: Detection of Agonist Activities of Compounds Against THRα and THRβ Based on Reporter Gene Activity Assay Method

### 1. Method

### 1.1 Construction and preparation of plasmids pGAL4-THR-LBD and pG5-Luc

The pGAL4-THRα-LBD and pGAL4-THRβ-LBD plasmids used in the reporter gene assay system were constructed by a conventional molecular cloning method comprising the following main steps: the cDNA sequences of THRα (NM_003250) and THRβ (NM_000461) corresponding to the amino acid sequences of THRα (163-407AA) and THRβ (217-461AA) were inserted into the BamHI and NotI enzyme digestion sites of the pGAL4 vector by the PCR technique respectively to obtain the pGAL4-THRα-LBD and pGAL4-THRβ-LBD plasmids; the pG5-Luc (#E249A) and pRL-TK (#E2241) plasmids were purchased from Promega; the plasmids were transfected into the *E. coli* DH5α by the CaCl₂ method, and after further culture and amplification, the plasmids were purified by Plasmid Extraction Kits (TIANGEN, #D107) to obtain the corresponding plasmid DNAs.

### 1.2 Co-transfection of plasmids into HEK293T cells and treatment with compounds

The HEK293T cells were inoculated into a 96-well plate at a density of 1×10⁴/well the day before plasmid transfection. Cell transfection was conducted according to the instructions of the transfection reagent FuGENE^{®} HD (Promega, # E2311). The main steps were as follows: taking one well as an example here, the plasmids pGAL4-THRα-LBD or pGAL4-THRβ-LBD, pG5-Luc, and pRL-TK were added in the proportions of 20 ng, 50 ng, and 5 ng to 10 µL of Opti-MEM^{™} I medium (Gibco, #11058021) and mixed well; subsequently, 0.25 µL of FuGENE^{®} HD was added, mixed well, and then left at room temperature for 5 min; afterwards, 10 µL of the mixture was added to the cell wells containing 100 µL of culture medium. After cell co-transfection for 6 h, the compound was diluted in a 3-fold gradient with dimethyl sulfoxide at the highest concentration of 1 µM. The compound was added to the cell culture medium at a total of 10 concentrations for treatment for 24 h. There were a total of two duplicate wells for each concentration. Triiodothyronine (T3) was used as the positive control.

### 1.3 Dual-Glo Luciferase Assay

After the cells were treated with the compound for 24 h, the cells were assayed according to the instructions of Dual-Glo^{®} Luciferase Assay System (Promega, # E2940). The main steps were as follows: 50 µL of culture medium was aspirated from each well and discarded, and then 50 µL of Dual-Glo^{®} Luciferase reagent was added and oscillated at room temperature for 10 min; 80 µL of lysis reaction solution was measured into a white opaque optiPlate-96-well plate, and the luminescence signal value (Firefly-Luc) of the Firefly luciferase was detected using an MD i3x multi-mode microplate reader; 40 µL of Dual-Glo^{®} Stop & Glo^{®} reagent was then added, and oscillated at room temperature for 10 min; the luminescence signal value (Renilla-Luc) of the Renilla luciferase was then detected using an MD i3x multi-mode microplate reader. The Firefly-Luc/Renilla-Luc ratio was used as the activation activity of the compound against THR, and was normalized against the ratio in the vehicle DMSO group; the dose-response curve was fitted with four parameters using the GraphPad Prism6.0 software, and the EC50 value was calculated.

### 2. Results

The experimental data suggested that the compounds of the present disclosure exhibited a stronger THRβ agonist activity and certain THRα/β selectivity. The specific data were listed in Table 2.

**Table 2**

| **Name of Compound** | THRα Agonist Activity EC50 (µM) | THRβ Agonist Activity EC50 (µM) | Selectivity (THRα/THRβ) |
|---|---|---|---|
| **MGL3196** | * | *** | † |
| **Compound 1** | *** | **** | † |
| **Compound 2** | **** | **** | † |
| **Compound 3** | **** | **** | † |
| **Compound 5** | **** | **** | † |
| **Compound 6** | *** | **** | † |
| **Compound 7** | **** | **** | † |
| **Compound 8** | ** | **** | † |
| **Compound 12** | ** | **** | † |
| **Compound 13** | **** | **** | † |
| **Compound 14** | ***** | ***** | † |
| **Compound 17** | **** | ***** | ††† |
| **Compound 18** | ***** | ***** | †† |
| **Compound 21** | **** | ***** | † |
| **Compound 27** | ** | **** | † |
| **Compound 28** | * | **** | †††† |
| **Compound 29** | * | ***** | †††† |
| **Compound 30** | * | **** | †††† |
| **Compound 32** | **** | ***** | † |
| **Compound 35** | **** | ***** | † |
| **Compound 37** | * | **** | †††† |
| **Compound 41** | ***** | ***** | † |
| **Compound 43** | ***** | ***** | † |
| **Compound 44** | ***** | ***** | † |
| **Compound 45** | ***** | ***** | † |
| **Compound 46** | ***** | ***** | † |
| **Compound 47** | **** | ***** | † |
| **Compound 48** | **** | ***** | † |
| **Compound 49** | **** | ***** | † |
| **Compound 51** | ***** | ***** | † |
| **Compound 71** | * | **** | †††† |
| **Compound 72** | * | **** | ††† |
| **Compound 73** | NA | NA | NA |

| | | | |
|---|---|---|---|
| *: EC50 > 20 µM; **: 20 µM ≥ EC50 > 10 µM; ***: 10 µM ≥ EC50 > 5 µM; ****: 5µM ≥ EC50 > 1 µM; *****: 1 µM ≥ EC50 †: 5 ≥ THRα/β; ††: 10 ≥ THRα/β > 5; †††: 20 ≥ THRα/β > 10; ††††: THRα/β > 20 NA: No activity | | | |

## Claims

1. A compound having a structure of formula (1) or a pharmaceutically acceptable salt thereof: 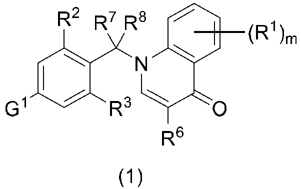 wherein
G¹ is 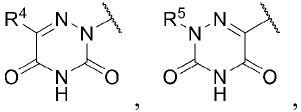 -O-(C₁₋₆ alkylene)-G², -NH-(C₁₋₆ alkylene)-G², -NHCO-(C₁₋₆ alkylene)-G² or -NHCO-G²;
G² is -COOH, -P(=O)(OH)₂, -P(-O)(OC₁₋₆ alkyl)₂, 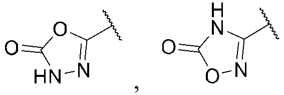 or 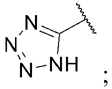
R⁴ and R⁵ are independently H, halogen, -CN, -NH₂, -NO₂, -OH, C₁₋₆ alkyl, -O(C₁₋₆ alkyl), -COO(C₁₋₆ alkyl), C₂₋₆ alkenyl, C₂₋₆ alkynyl or C₃₋₈ cycloalkyl; said C₁₋₆ alkyl, C₂₋₆ alkenyl, C₂₋₆ alkynyl or C₃₋₈ cycloalkyl is optionally substituted with one or more substituents that are independently deuterium, halogen, -CN, -NH₂, -NO₂ or -OH;
R² and R³ are independently H, halogen, -CN, -NH₂, -NO₂, -OH or C₁₋₆ alkyl; said C₁₋₆ alkyl is optionally substituted with one or more substituents that are independently halogen, -CN, -NH₂, -NO₂ or -OH;
R⁶ is H, -COOH, -COO(C₁₋₆ alkyl), -CONH(C₁₋₆ alkyl) or -CONH(C₃₋₈ cycloalkyl); said C₁₋₆ alkyl or C₃₋₈ cycloalkyl is optionally substituted with one or more substituents that are independently halogen, -CN, -NH₂, -NO₂ or -OH;
R⁷ and R⁸ are independently H or deuterium;
each R¹ is independently H, halogen, -CN, -NH₂, -NO₂, -OH, C₁₋₆ alkyl, -O(C₁₋₆ alkyl) or -COO(C₁₋₆ alkyl); said C₁₋₆ alkyl is optionally substituted with one or more substituents that are independently halogen, -CN, -NH₂, -NO₂ or -OH; and
m is 0, 1, 2, 3 or 4.

2. The compound or the pharmaceutically acceptable salt thereof according to claim 1, wherein
G¹ is 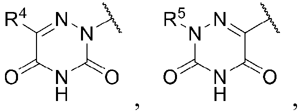 -O-(C₁₋₃ alkylene)-G², -NH-(C₁₋₃ alkylene)-G², -NHCO-(C₁₋₃ alkylene)-G² or -NHCO-G²; and
G² is -COOH, -P(=O)(OH)₂, -P(=O)(OC₁₋₃ alkyl)₂, 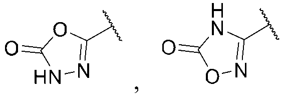 or 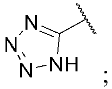
preferably, G¹ is

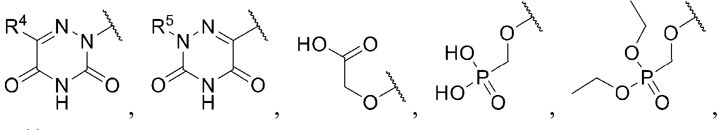

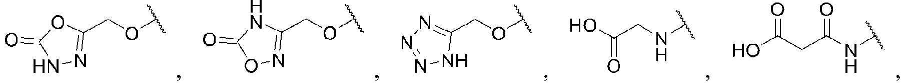

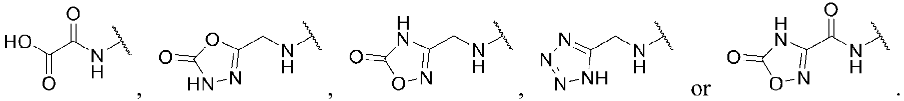

3. The compound or the pharmaceutically acceptable salt thereof according to claim 1 or 2, wherein
R⁴ and R⁵ are independently H, halogen, -CN, -NH₂, -NO₂, -OH, C₁₋₃ alkyl, -O(C₁₋₃ alkyl), -COO(C₁₋₃ alkyl), C₂₋₄ alkenyl, C₂₋₄ alkynyl or C₃₋₆ cycloalkyl; said C₁₋₃ alkyl, C₂₋₄ alkenyl, C₂₋₄ alkynyl or C₃₋₆ cycloalkyl is optionally substituted with one or more substituents that are independently deuterium, halogen, -CN, -NH₂, -NO₂ or -OH;
preferably, R⁴ and R⁵ are independently H, -CN, -NH₂, -CH₃, -CH₂CH₃, -CH₂F, -CHF₂, -CDF₂, -CF₃, -COOCH₃, -CH₂OH, -CH=CH₂, -OCH₃, -C≡CH or

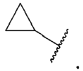

4. The compound or the pharmaceutically acceptable salt thereof according to any one of claims 1 to 3, wherein
R² and R³ are independently H, F, Cl, Br, -CN, -NH₂ or C₁₋₃ alkyl; said C₁₋₃ alkyl is optionally substituted with one or more substituents that are independently F, Cl, Br, -CN, -NH₂, -NO₂ or -OH;
preferably, R² and R³ are independently H, F, Cl, Br or -CH₃,

5. The compound or the pharmaceutically acceptable salt thereof according to any one of claims 1 to 4, wherein
R⁶ is H, -COOH, -COO(C₁₋₃ alkyl), -CONH(C₁₋₃ alkyl) or -CONH(C₃₋₆ cycloalkyl); said C₁₋₃ alkyl or C₃₋₆ cycloalkyl is optionally substituted with one or more substituents that are independently halogen, -CN, -NH₂, -NO₂ or -OH;
preferably, R⁶ is H, -COOH, -COOCH₃, -COOCH₂CH₃, -CONHCH₃, 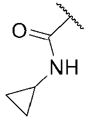
or

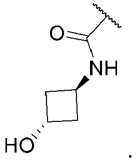

6. The compound or the pharmaceutically acceptable salt thereof according to any one of claims 1 to 5, wherein
each R¹ is independently H, halogen, -CN, -NH₂, -NO₂, -OH, C₁₋₃ alkyl, -O(C₁₋₃ alkyl) or -COO(C₁₋₃ alkyl); said C₁₋₃ alkyl is optionally substituted with one or more substituents that are independently halogen, -CN, -NH₂, -NO₂ or -OH;
preferably, each R¹ is independently H, F, Cl, Br, -CN, -CH₃, -CF₃, -OCH₃, -OCF₃ or -COOCH₃.

7. A compound or a pharmaceutically acceptable salt thereof, wherein the compound is selected from the group consisting of:

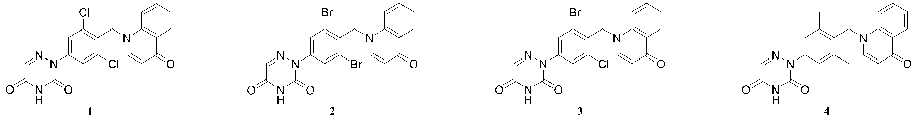

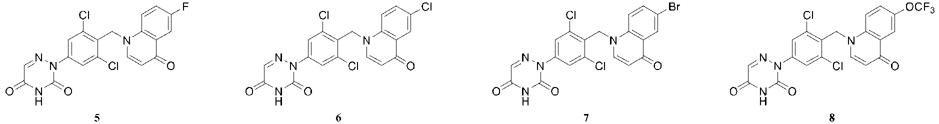

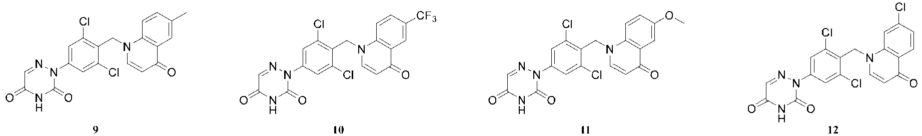

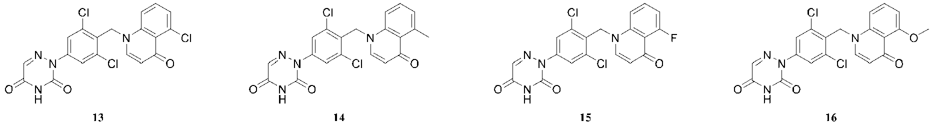

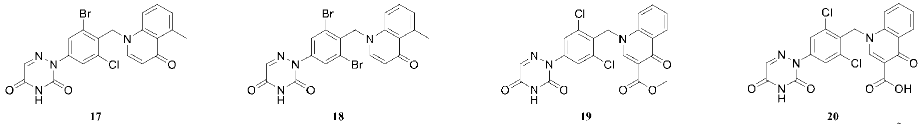

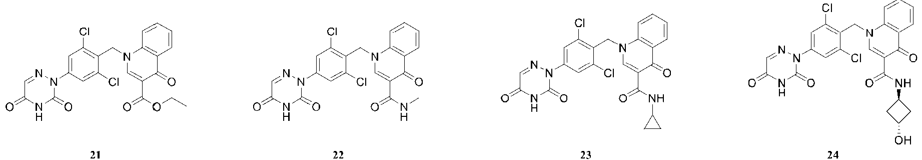

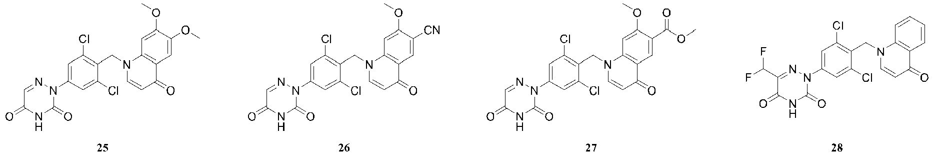

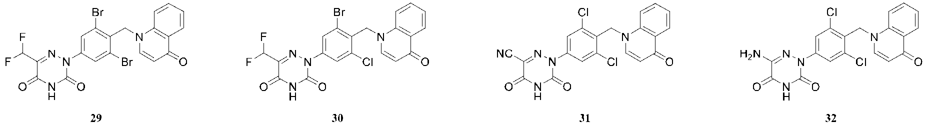

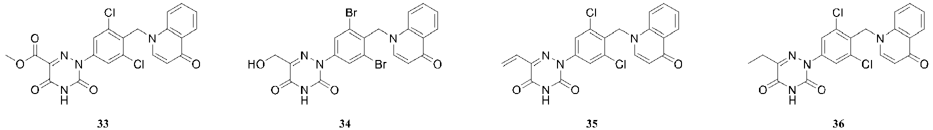

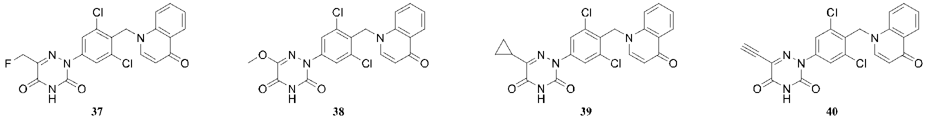

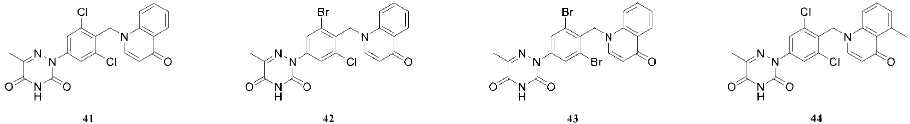

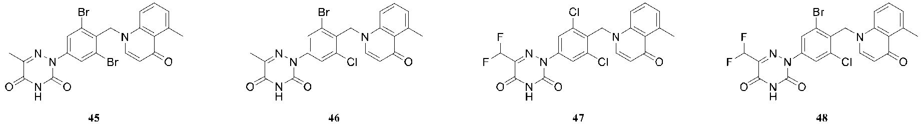

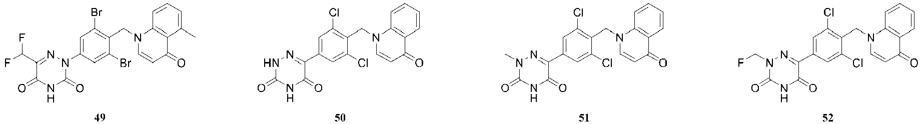

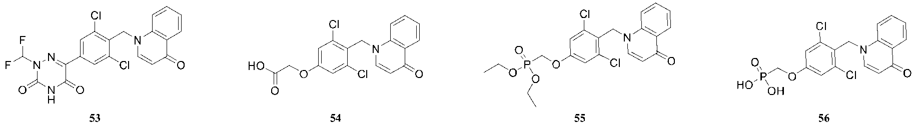

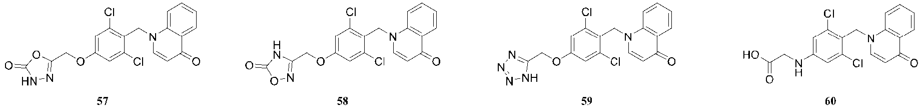

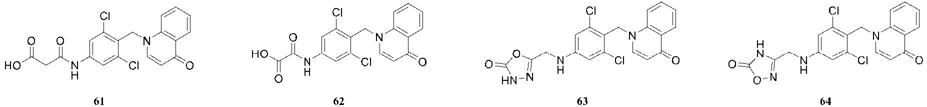

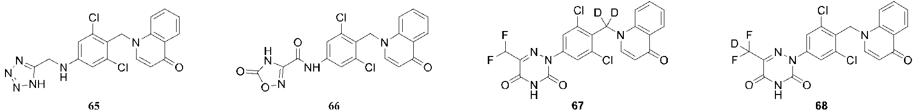

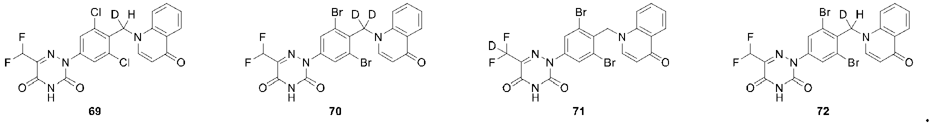

8. A pharmaceutical composition, comprising the compound or the pharmaceutically acceptable salt thereof according to any one of claims 1 to 7, and one or more pharmaceutically acceptable carriers.

9. Use of the compound or the pharmaceutically acceptable salt thereof according to any one of claims 1 to 7, or the pharmaceutical composition according to claim 8 in the preparation of a medicament for preventing or treating a disease mediated by thyroid hormone β receptor.

10. The use according to claim 9, wherein the disease is a metabolic disease.

11. The use according to claim 10, wherein the disease is nonalcoholic fatty liver disease, dyslipidemia, atherosclerosis, or hypothyroidism.
